# DEMANDE DE BREVET EUROPEEN

(11) **EP 3 865 114 A1**
(43) Date de publication de la demande: **18.08.2021**
(21) Numéro de dépôt: 21155724.4
(22) Date de dépôt: 08.02.2021
(51) Int. Cl.: A61K 8/04, A61K 8/73, A61K 8/9711, A61Q 19/00, A61K 8/81

(54) **COMPOSITION À USAGE TOPIQUE SE PRÉSENTANT SOUS LA FORME D'UN GEL COMPRENANT UNE EAU MINÉRALISÉE**

(30) Priorité: 12.02.2020 FR 2001376
(71) Demandeur: Société d'Exploitation de Produits pour les Industries Chimiques SEPPIC, 75321 Paris cedex 07 (FR)
(72) Inventeur: SIGURANI, Séverine, 81100 Castres (FR); TAILLEBOIS, Cécile, 81100 Castres (FR)
(74) Mandataire: Air Liquide

(57) **Abrégé**

Composition à usage topique (C1) se présentant sous la forme d'un gel et comprenant:
- de l'eau dont la conductivité mesurée à 25°C est supérieure ou égale à 300 micro-siemens par centimètre (ou µS.cm⁻¹)
- un polyélectrolyte anionique réticulé (P1) et
- au moins un galactomannane (GM).

L'eau est de préférence une eau minérale naturelle, une eau thermale, une eau de mer ou une eau d'algues. Le polyélectrolyte anionique réticulé peut comprendre des unités monomériques issues de l'acide 2-méthyl 2-[(1-oxo 2-propènyl) amino] 1-propanesulfonique sous forme acide libre ou partiellement ou totalement salifiée. Une formulation (F) à usage topique comprend la composition C1 et une phase grasse (A2) comprenant i) au moins une huile, et ii) un système émulsionnant comprenant au moins un agent tensioactif émulsionnant (S1).

## Description

La présente invention est relative à une composition à usage topique se présentant sous la forme d'un gel, comprenant une eau de minéralisation a minima moyenne, par conséquent pour une conductivité, mesurée à 25°C, supérieure ou égale à 200 µS/cm, et d'un système épaississant performant.

Les industries de la cosmétique utilisent des eaux de diverses qualités pour préparer les formulations cosmétiques qu'elles proposent à la vente au consommateur final. On peut ainsi citer les eaux minérales naturelles, les eaux de mers, les eaux thermales, les eaux de végétaux terrestres, les eaux d'algues.

Ces différentes eaux se distinguent notamment par leur origine géographique et géologique, et par conséquent par leurs caractéristiques minérales. On peut ainsi caractériser une eau par sa « minéralisation totale », à savoir par la somme de tous les éléments minéraux présents dans l'eau considérée. Il s'agit plus particulièrement de la somme de la quantité de cations et de la quantité d'anions présentes dans ladite eau. De façon plus particulière, les cations compris dans une eau minérale sont les cations du calcium, du magnésium, du sodium, du potassium, du fer, du zinc, du cuivre, de l'aluminium, du manganèse. De façon plus particulière, les anions compris dans une eau minérale sont les anions carbonates, hydrogéno-carbonates, silicates, sulfates, chlorures, nitrates, phosphates et fluorures. Par conséquent, la notion de « minéralisation totale » s'exprime en unité de masse par unité de volume, notamment en milligramme/litre.

Cette caractéristique de minéralisation peut également être retranscrite par une mesure de conductivité électrique de ladite eau, qui s'exprime généralement en micro-Siémens par centimètre (µS/cm) ou en milliSiémens/cm (mS/cm).

Dans le cadre de la présente invention, par eau de faible minéralisation, on entendra une eau dont la conductivité, mesurée à 25°C, est inférieure à 200 µS/cm.

Dans le cadre de la présente invention, par eau de moyenne minéralisation, on entendra une eau dont la conductivité, mesurée à 25°C, est supérieure ou égale à 200 µS/cm et inférieure à 333 µS/cm.

Dans le cadre de la présente invention, par eau de minéralisation moyenne accentuée, on entendra une eau dont la conductivité, mesurée à 25°C, est supérieure ou égale à 333 µS/cm et inférieure à 666 µS/cm.

Dans le cadre de la présente invention, par eau de minéralisation importante, on entendra une eau dont la conductivité, mesurée à 25°C, est supérieure ou égale à 666 µS/cm et inférieure à 1000 µS/cm.

Dans le cadre de la présente invention, par eau de minéralisation élevée, on entendra une eau dont la conductivité, mesurée à 25°C, est supérieure ou égale à 1000 µS/cm.

Les eaux minérales naturelles, les eaux de mer, les eaux thermales, les eaux d'algues qui se caractérisent par une minéralisation *a minima* moyenne, par conséquent par une conductivité, mesurée à 25°C, supérieure ou égale à 200 µS/cm, sont fréquemment utilisées pour la préparation de compositions cosmétiques, dermo-cosmétiques, pharmaceutiques et dermo-pharmaceutique pour les propriétés qu'elles peuvent apporter à la peau humaine ou animale.

En effet, il est connu dans la littérature que les eaux thermales, les eaux minérales, les eaux de mer, les eaux d'algues de minéralisation moyenne à minéralisation élevée, sont utilisées dans des formulations pour leur contribution à conférer des propriétés anti-oxydantes, des propriétés apaisantes ou anti-inflammatoires, des propriétés inhibitrices d'allergies. Ainsi, la demande de brevet publiée sous le numéro EP 1170002 A1 décrit une méthode pour réduire la perte d'élasticité de la peau humaine qui inclut l'utilisation d'une eau minérale contenant une concentration d'eau moins 200 mg/l de minéraux. De même, le brevet publié sous le numéro EP0699432 B1 décrit l'utilisation d'une eau dont la minéralisation totale est supérieure ou égale à 700 mg/l pour apaiser les irritations de la peau que pourrait apporter des agents actifs tels que les rétinoïdes, le péroxyde de benzoyle ou l'acide salicylique utilisés pour le traitement de l'acné.

Il est également connu que l'utilisation d'eau d'algues ou d'eau de mer peuvent être utilisées pour le soin de maladies de la peau, comme par exemple l'eczéma et le psoriasis, ou pour la remise en forme après des traumatismes ou des pathologies musculo tendineuses ou des entorses.

Il existe donc un besoin avérer de procéder à des applications locales et prolongées d'eau de mer ou d'eau d'algues, en présence ou non d'un principe biologiquement actif ou non, pour mieux cibler la zone du corps humain ou animal concerné.

La demande de brevet japonais publiée sous le numéro JP 2000-273033 décrit des compositions sous forme de gel, comprenant des principes actifs, et une combinaison de tréhalose et de carraghénanes comme agents gélifiants.

Le brevet européen publié sous le numéro EP 2756839B1 décrit une composition à base de 95% massique d'eau de mer et d'hydroxyéthyl méthyl cellulose comme agent gélifiant.

Cependant, ces solutions ne permettent pas de préparer des compositions topiquement acceptables se présentant sous forme de gel de viscosités élevées, comme par exemple supérieure ou égale à 10 000 mPa.s (mesurée à 25°C, avec un viscosimètre Brookfield RVT, Vitesse 5).

Les compositions cosmétiques se présentant sous la forme de gels aqueux commercialisés par l'industrie cosmétique et par l'industrie pharmaceutique comprennent très régulièrement des polymères épaississants synthétiques pour augmenter la viscosité des solutions de façon à atteindre des gels, qui sont appliqués directement ou indirectement sur la peau.

Les polymères épaississants synthétiques actuellement utilisés en ces domaines, se présentent sous deux formes physiques, la forme poudre et la forme liquide pour laquelle le polymère est préparé par polymérisation radicalaire en émulsion inverse à l'aide d'agents tensioactifs, et que l'on appelle couramment latex inverses auto-inversibles.

Parmi les polymères épaississants synthétiques se présentant sous la forme d'une poudre les plus connus, on peut citer les polymères à base d'acide acrylique ou les copolymères à base d'acide acrylique et de ses esters. On citera par exemple, les polymères commercialisés sous le nom de marque, CARBOPOL™ et PEMULEN™. Ils sont décrits notamment dans les brevets américains US 5,373,044, US 2,798,053 et dans le brevet européen EP 0 301 532B1. Ils présentent comme inconvénients de devoir être dispersés, puis d'être neutralisés par ajout d'une base pour atteindre le taux de salification souhaité, corrélée avec la viscosité souhaitée.

En cosmétique, on utilise aussi et toujours sous forme de poudre, des homopolymères ou des copolymères à base d'acide 2-acrylamido-2-methyl-propane sulfonique et/ou de ses sels. Ces polymères épaississants sont commercialisés sous le nom de marque Aristoflex™ et décrits notamment dans les brevets européens EP 816 403, EP 1116 733 et EP 1 069 142. Ces épaississants synthétiques sous forme de poudre sont obtenus par polymérisation précipitante; le (ou les) monomère(s) est (ou sont) mis en solution dans un solvant organique de type benzène, acétate d'éthyle, cyclohexane, tertio-butanol; ce procédé nécessite donc de nombreuses étapes successives de purification du produit final, pour éliminer toute trace de solvant résiduel.

Les industries de la cosmétique et de la pharmacie utilisent aussi très largement des épaississants se présentant sous la forme liquide de latex inverses auto-inversibles et notamment ceux commercialisés par la demanderesse. On citera par exemple, les épaississants Sepigel™ 305, Simulgel™ 600, Simulgel™ EG, Simulgel™ EPG, Simulgel™ NS, Simulgel™ A, Sepiplus™400, Sepiplus™250 et Sepiplus™265. Ces épaississants sont obtenus par polymérisation radicalaire en émulsion inverse. Ils présentent l'avantage d'être plus aisément manipulables, notamment à température ambiante, et se dispersent très rapidement dans l'eau. De plus, ces produits développent des performances épaississantes remarquablement élevées; ces performances sont probablement la conséquence du procédé mis en œuvre pour leur préparation, une réaction de polymérisation radicalaire en phase dispersée, qui conduit à des polymères de très hauts poids moléculaires.

La demanderesse a également développé des épaississants synthétiques ayant les performances épaississantes équivalentes ou supérieures aux latex inverses, mais mieux tolérés par la peau, en particulier du fait de l'absence de toute phase huile pouvant conduire à des gels aqueux plus clairs. Ces produits se présentent sous la forme de poudre mais possèdent des temps de dissolution, et donc une facilité de mise en œuvre, comparable à ceux des produits se présentant sous la forme de liquides. Ces composés, décrits dans la demande de brevet européen publiée sous le numéro EP 1496 081, sont obtenus par les techniques classiques de polymérisation, telles que la polymérisation radicalaire en phase dispersée, la polymérisation radicalaire en suspension inverse, la polymérisation radicalaire en émulsion inverse ou en microémulsion inverse. Les systèmes épaississants synthétiques obtenus sont ensuite extraits et purifiés par différentes techniques telles que la précipitation dans un tiers solvant, la précipitation dans un tiers solvant, suivie éventuellement d'un lavage, d'un séchage par atomisation ou par déshydratation azéotropique, suivie éventuellement d'un lavage par un solvant judicieusement choisi. Ces épaississants synthétiques combinent donc une partie des avantages des épaississants synthétiques se présentant sous la forme de poudres classiques (absence d'huile, obtention de gels aqueux plus clairs) et les avantages des épaississants synthétiques se présentant sous la forme de latex inverse (haute vitesse de dissolution, pouvoir épaississant et propriétés stabilisantes remarquables).

Cependant pour des utilisations en présence d'eau de minéralisation a minima moyenne, par conséquent pour une conductivité, mesurée à 25°C, supérieure ou égale à 200 µS/cm, les polymères épaississants synthétiques, tels que les polyélectrolytes anioniques réticulés tels que décrits précédemment, ne sont pas jugés suffisamment performants.

Il existe donc toujours un besoin de disposer d'une composition à usage topique se présentant sous la forme d'un gel, comprenant une eau de minéralisation a minima moyenne, par conséquent pour une conductivité, mesurée à 25°C, supérieure ou égale à 200 µS/cm, et d'un système épaississant performant, à savoir permettant d'atteindre notamment des niveaux de viscosité élevés pour des applications localisées sur la peau humain ou animale optionnellement en présence d'un agent biologiquement actif.

Une solution de la présente invention est une composition à usage topique (C1) se présentant sous la forme d'un gel et comprenant:
- de l'eau dont la conductivité mesurée à 25°C est supérieure ou égale à 300 micro-siemens par centimètre (ou µS.cm⁻¹)
- un polyélectrolyte anionique réticulé (P1) et
- au moins un galactomannane (GM).

Dans le cadre de la présente invention, on désigne par « gel » une composition chimique se présentant initialement à l'état liquide qui, après ajout de substances gélifiantes et/ou épaississantes, est transformé en un état structuré, qui ne coule pas. Un gel tel que défini, est considéré comme un état intermédiaire entre l'état solide et l'état liquide, et est constitué par un réseau tridimensionnel au sein du liquide étant le résultat de liaisons chimiques ou physiques.

Par « agent gélifiant », on entend au sens de la présente invention un composé chimique ou un mélange de composés chimiques, qui transforme un milieu liquide en un gel.

Par « agent épaississant », on entend au sens de la présente invention un composé chimique ou un mélange de composés chimiques, qui augmente la viscosité du milieu dans lequel il est introduit.

L'expression "à usage topique" utilisée dans la définition de la composition (C₁) telle que décrite ci-dessus, signifie que ladite composition est mise en œuvre par application sur la peau, les cheveux, le cuir chevelu, les ongles, les lèvres, les muqueuses, les cils, les sourcils, qu'il s'agisse d'une application directe dans le cas d'une formulation cosmétique ou d'une application indirecte par exemple dans le cas d'un produit de soin corporel sous forme de lingette en textile ou en papier ou de produits sanitaires destinés à être en contact avec la peau, les cheveux, le cuir chevelu, les ongles, les lèvres, les muqueuses, les cils, les sourcils. La mesure de la conductivité de la phase aqueuse (A1) est réalisée à une température de 25°C par une mesure directe du courant passant entre deux électrodes immergées dans la phase aqueuse (A1) à mesurer. Le courant injecté est un courant alternatif de très basse fréquence afin d'éviter la polarisation des électrodes.

L'appareillage utilisé se compose d'un boîtier d'acquisition et d'une sonde. La sonde est constituée d'une électrode de température, d'une électrode de mesure constituée de deux ou plusieurs capteurs ou d'un dispositif d'électrodes groupées.

L'appareillage utilisé est plus particulièrement un appareil de la marque WTW, modèle pH/Cond 34Oi.

Les mesures sont exprimées en µS/cm à la température de référence (25 °C).

La phase aqueuse (A1) présente dans la composition à usage topique (C₁) objet de la présente invention, et se caractérisant par une conductivité mesurée à 25°C supérieure ou égale à 300 µS.cm⁻¹, peut provenir de sources naturelles et être utilisée seule ou en mélange avec une eau d'une autre origine.

La phase aqueuse (A1) présente dans la composition à usage topique (C₁) objet de la présente invention, et se caractérisant par une conductivité mesurée à 25°C supérieure ou égale à 300 µS.cm⁻¹, est de préférence constituée par une eau minérale naturelle, une eau thermale, une eau de mer, ou une eau d'algues.

La phase aqueuse (A1) présente dans la composition à usage topique (C₁) objet de la présente invention, et se caractérisant par une conductivité mesurée à 25°C supérieure ou égale à 300 µS.cm⁻¹, peut également être préparée par le mélange d'une eau minérale naturelle, une d'eau thermale, d'une eau de mer, ou d'une eau d'algues, avec une eau déminéralisée de sorte à régler la valeur de la conductivité retenue.

Par « eau minérale naturelle », on désigne au sens de la présente invention une eau dont la « minéralisation totale », la teneur en oligo-éléments est stable et propre à la consommation, répond aux exigences de la définition de l'article R1322-2 du Code de la santé publique, et plus généralement aux dispositions des articles L.1322-1 à L132-13, R.1322-1 à R.1322-44-8, R. 1322-45 à R.1322-67 du Code de la santé publique.

Par « eau thermale », on désigne au sens de la présente invention une eau minérale naturelle telle que définie précédemment qui est exploitée à des fins thérapeutiques et fait l'objet d'une autorisation préalable par les autorités préfectorales françaises après instruction par l'Agence Régionale de Santé.

Comme exemples d'eaux thermales ou d'eaux minérales naturelles que l'on peut associer à la phase aqueuse (A1) présente dans la composition (C₁) selon l'invention, on peut citer par exemple l'eau d'Avène, l'eau de Vittel, les eaux du bassin de Vichy, l'eau d'Uriage, l'eau de la Roche-Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizieres, l'eau de Neyrac-les-bains, l'eau de Lons le Saunier, l'eau de Rochefort, l'eau de Saint Christau, l'eau des Fumades, l'eau de Tercis-les-bains, l'eau de Bagnères-de -Bigorres, l'eau d'Eugénie-les-Bains, l'eau de Chlles-les-eaux, l'eau de Volvic, l'eau de Vals, l'eau de Vernière, l'eau d'Aix les Bains, l'eau d'Alet, l'eau d'Abatilles, l'eau d'Arcens, l'eau d'Arvie, l'eau d'Asperjoc, l'eau de Badoit, l'eau de Cilaos, l'eau de Contrex, l'eau d'Evian, l'eau d'Hépar, l'eau de Jouvence, l'eau du Mont-Roucous, l'eau d'Ogeu, l'eau d'Orezza, l'eau de Parot, l'eau de Perrier, l'eau de Plantcoët, l'eau de Quézac, l'eau de Rozana, l'eau de Saint-Albans, l'eau de Saint-Amand-les Eaux, l'eau de Saint-Georges, l'eau de Saint-Géron, l'eau de Sainte-Marguerite, l'eau de Saint-Yorre, l'eau de la Salvetat, l'eau de Teissières-lès-Bouliès, l'eau de Thonon, l'eau de Treignac, l'eau de Courmayeur, l'eau de San Benedetto, l'eau de San Pellegrino.

Par « eau d'algues », on désigne au sens de la présente invention une eau recueillie par évaporation et condensation de l'eau comprise dans les algues après récolte, ladite eau provenant des cellules des algues ainsi traitées. Selon un aspect particulier, il s'agit d'eau d'algues brunes, d'eau d'algues rouges, d'eau d'algues vertes. Selon un aspect encore plus particulier, il s'agit d'eau de *Laminaria digitata,* d'eau de *Chondrus crispus, d'eau de Gigartina stellata.*

Comme exemples d'eaux d'algues que l'on peut associer à la phase aqueuse (A1) présente dans la composition (C₁) selon l'invention, on peut citer par exemple l'eau d'algues commercialisées sous le nom de marque Hydralixir™ LD ou Hydralixir™CC.

Par « galactomannane (GM) » on désigne au sens de la présente invention, les polysaccharides constitués uniquement d'oses (ou poly-oses), qui sont des polymères dont la chaîne principale est constituée d'unités de D-mannose, reliées entre elles en β-1,4, et sur laquelle des unités de D-galactose sont greffées latéralement par des liaisons α-1,6.

Les polysaccharides sont des polymères de saccharides. La définition IUPAC des saccharides désigne des oses, des composés d'oses proprement dits et leurs dérivés obtenus soit par réduction d'un groupement carbonyle, soit par oxydation d'une ou plusieurs fonctions hydroxyles, soit par le remplacement d'une ou plusieurs fonctions hydroxyles par un atome d'hydrogène, par un groupement amine, une fonction phosphate, une fonction sulfate.

Les polysaccharides les plus couramment utilisés pour la préparation de compositions alimentaires, cosmétiques ou pharmaceutiques, sont majoritairement constitués d'oses, tels que le glucose, le galactose, le mannose ou de dérivés d'oses pour lesquels la fonction hydroxyle du carbone terminal a été oxydée en fonction carboxyle. Par conséquent, on peut distinguer parmi les polysaccharides deux groupes distincts : les polysaccharides constitués uniquement d'oses (ou poly-oses) et les polysaccharides constitués de dérivés d'oses. Parmi les polysaccharides composés uniquement d'oses, on peut distinguer :
- les glucanes, qui sont des homopolymères de glucose très abondants dans la nature,
- les glucomannoglycanes,
- les xyloglycanes,
- les galactomannanes, qui sont des polymères dont la chaîne principale est constituée d'unités de D-mannose, reliées entre elles en β-1,4, et sur laquelle des unités de D-galactose sont greffées latéralement par des liaisons α-1,6.

Les galactomannanes se rencontrent dans plusieurs espèces végétales, et plus particulièrement dans les espèces légumineuses dans lesquelles ils constituent l'albumen des graines.

En fonction de l'origine végétale de la provenance des galactomannanes, le degré de substitution (DS) des unités de D-galactose sur la chaîne principale de D-mannose varie entre 0 et 1 :
- les galactomannanes provenant de la gomme de cassia présentent un degré de substitution (DS) d'environ 1/5, signifiant le greffage latéral d'une unité de D-galactose toutes les 5 unités de D-mannose présentes sur la chaîne principale du polysaccharide
- les galactomannanes provenant de la gomme de caroube présentent un degré de substitution (DS) d'environ 1/4, signifiant le greffage latéral d'une unité de D-galactose toutes les 4 unités de D-mannose présentes sur la chaîne principale du polysaccharide
- les galactomannanes provenant de la gomme de tara présentent un degré de substitution (DS) d'environ 1/3, signifiant le greffage latéral d'une unité de D-galactose toutes les 3 unités de D-mannose présentes sur la chaîne principale du polysaccharide
- les galactomannanes provenant de la gomme de guar présentent un degré de substitution (DS) d'environ 1/2, signifiant le greffage latéral d'une unité de D-galactose toutes les 2 unités de D-mannose présentes sur la chaîne principale du polysaccharide
- les galactomannanes provenant de la gomme de fenugrec présentent un degré de substitution (DS) d'environ 1/1, signifiant le greffage latéral d'une unité de D-galactose pour quasiment toutes les unités de D-mannose présentes sur la chaîne principale du polysaccharide

Ainsi dans la composition selon l'invention, le galactomannane (GM) est choisi parmi les éléments du groupe constitué par les galactomannanes présentant un degré de substitution (DS) d'environ 1/3, les galactomannanes présentant un degré de substitution (DS) d'environ 1/2, les galactomannanes présentant un degré de substitution (DS) d'environ 1/4.

Par polyélectrolyte anionique réticulé (P1), on désigne, dans la définition de la composition (C1) à usage topique objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Selon le cas, la composition selon l'invention peut présenter une ou plusieurs des caractéristiques ci-dessous :
- la composition comprend pour 100% de sa masse : de 93% à 99,4% massique, de préférence de 95% à 99,3% massique, et encore plus préférentiellement de 95,5% à 99,0% massique de l'eau dont la conductivité mesurée à 25°C est supérieure ou égale à 300 micro-siemens par centimètre (ou µS.cm⁻¹), de 0,5 à 5% massique, de préférence de 0,5% à 3%, et encore plus préférentiellement de 0,5% à 2,5% massique du polyélectrolyte anionique réticulé (P1) et de 0,1 à 2% massique, de préférence de 0,2% à 2%, et encore plus préférentiellement de 0,5% à 2% massique du galactomannane (GM).
- le galactomannane présente un degré de substitution d'1/3.
- le polyélectrolyte anionique réticulé (P1) comprend des unités monomériques issues de l'acide 2-méthyl 2-[(1-oxo 2-propènyl) amino] 1-propanesulfonique sous forme acide libre ou partiellement ou totalement salifiée. Au sens de la présente invention, le terme « salifié » indique que la fonction acide présente dans un monomère se trouve sous une forme anionique associée sous forme de sel à un cation, notamment les sels de métaux alcalins, tels que les cations du sodium ou du potassium, ou comme les cations de base azotés tels que le sel d'ammonium, le sel de lysine ou le sel de monoéthanolamine (HOCH₂-CH₂-NH₃⁺). Il s'agit de préférence des sels de sodium ou d'ammonium.
- le polyélectrolyte anionique réticulé (P1) est constitué d'une unité monomérique issue d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée ; et d'au moins une unité monomérique issue d'un monomère de réticulation (AR) polyéthylénique.
- le polyélectrolyte anionique réticulé (P1) est constitué d'une unité monomérique issue de l'acide acrylique et d'au moins une unité monomérique issue d'un monomère de réticulation (AR) polyéthylénique.
- le monomère de réticulation (AR) polyéthylénique est choisi parmi les éléments du groupe constitué par le méthylène-bis(acrylamide), le diméthacrylate d'éthylèneglycol, le diacrylate de diéthylèneglycol, le diacrylate d'éthylène glycol, le diallyl urée, le triallylamine, le triméthylol propanetriacrylate, l'acide diallyoxyacétique ou un de ses sels comme le diallyloxyacétate de sodium, ou un mélange de ces composés.
- le monomère de réticulation (AR) polyéthylénique est le triallylamine, le triméthylol propanetriacrylate ou le méthylène-bis(acrylamide) ou un mélange ces composés.
- ledit polyélectrolyte anionique réticulé (P1) est un élément du groupe constitué par un copolymère de l'acide 2-méthyl 2-[(1-oxo 2-propènyl) amino] 1-propanesulfonique (y) partiellement ou totalement salifié sous forme de sel de sodium, et de l'acide acrylique (δ) partiellement ou totalement salifié sous forme de sel de sodium dans un rapport molaire (γ)/(δ)supérieur ou égal à 40/60 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'hydroxyéthylacrylate (ζ) dans un rapport molaire (γ)/(ζ) supérieur ou égal à 30/70 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acrylamide (ε) dans un rapport molaire (γ)/(ε) supérieur ou égal à 30/70 et inférieur ou égal à 90/10, réticulé par le triallylamine et/ou le méthylène-bis(acrylamide).
- le ratio massique entre le galactomannane (GM) et le polyélectrolyte anionique réticulé (P1) est supérieur ou égal à 0,15 et inférieur ou égal à 0,75, de préférence supérieur ou égal à 0,20 et inférieur ou égal à 0,60, plus particulièrement supérieur ou égal à 0,25 et inférieur ou égal à 0,60, et encore plus préférentiellement supérieur ou égal à 0,20 et inférieur ou égal à 0,50.

Selon un aspect particulier, le polyélectrolyte anionique réticulé (P1) comprend pour 100% molaire :
(a₁) - une proportion supérieure ou égale à 25% molaire et inférieure ou égale à 100% molaire, d'unités monomériques issues de l'acide 2-méthyl 2-[(1-oxo 2-propènyl) amino] 1-propanesulfonique sous forme acide libre ou partiellement ou totalement salifiée ;
(a₂) - optionnellement, une proportion supérieure à 0% molaire et inférieure ou égale à 75% molaire, d'unités monomériques issues d'au moins un monomère choisi parmi les éléments du groupe constitué par l'acrylamide, le N,N-diméthyl acrylamide ; le méthacrylamide ; le tertiobutyl acrylamide ou le N-isopropyl acrylamide ;
(a₃) - optionnellement une proportion supérieure à 0% molaire et inférieure ou égale à 20% molaire, plus particulièrement supérieure à 0% molaire et inférieure ou égale à 15% molaire, encore plus particulièrement supérieure ou égale 0% molaire et inférieure ou égale à 10% molaire d'unités monomériques issues d'au moins un monomère choisi parmi les éléments du groupe constitué par l'acrylate de (2-hydroxy éthyle), l'acrylate de (2,3-dihydroxy propyle), le méthacrylate de (2-hydroxy éthyle), le méthacrylate de (2,3-dihydroxypropyle), et le vinyl pyrrolidone
(a₄) - optionnellement une proportion supérieure à 0% molaire et inférieure ou égale à 75% molaire, d'unités monomériques issues d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3- [(1-oxo 2-propènyl) amino] butanoïque, la fonction carboxylique desdits monomères étant sous forme acide libre, partiellement salifiée ou totalement salifiée ;
(a₅) optionnellement une proportion supérieure à 0% molaire et inférieure ou égale à 5% molaire d'au moins un monomère de formule (I) : 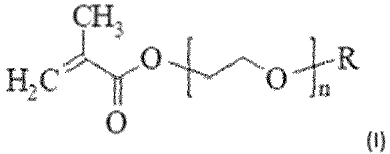 dans laquelle R représente un radical alkyle linéaire ou ramifié comportant de huit à vingt atomes de carbone et n représente un nombre entier supérieur ou égal à zéro et inférieur ou égal à vingt ;
(a₆) - d'une proportion supérieure à 0% molaire et inférieure ou égale à 1% molaire d'unités monomériques issues d'au moins un monomère de réticulation (AR) polyéthylénique ; la somme des dites proportions molaires en unités monomériques selon a1), a2), a3), a4), a5) et a6) étant égale à 100% molaire.

Selon un autre aspect particulier de la présente invention, la composition (C₁) à usage topique est caractérisée en ce que ledit monomère de réticulation (AR) polyéthylénique tel que défini précédemment, est mis en œuvre dans une proportion molaire inférieure ou égale à 0,5%, plus particulièrement inférieure ou égale à 0,25% et tout particulièrement inférieure ou égales à 0,1% ; elle est plus particulièrement supérieure ou égales à 0,005% molaire.

Le polyélectrolyte anionique réticulé (P1) mis en œuvre dans la composition (C₁) à usage topique objet de la présente invention peut également comprendre divers additifs, tels que des agents complexants, des agents de transfert ou des agents limiteurs de chaîne.

Le polyélectrolyte anionique réticulé (P1) mis en œuvre dans la composition (C₁) à usage topique objet de la présente invention peut également comprendre divers additifs, tels que des agents complexants, des agents de transfert ou des agents limiteurs de chaîne.

Le polyélectrolyte anionique réticulé (P1) mis en œuvre dans la composition (C₁) à usage topique objet de la présente invention peut être préparé par la mise en œuvre d'un procédé de polymérisation radicalaire connu de l'homme du métier, comme par exemple les procédés de polymérisation en solution, de polymérisation en suspension, de polymérisation en suspension inverse, de polymérisation en émulsion, de polymérisation en émulsion inverse, de polymérisation en milieu solvant suivie d'une étape de précipitation du polymère formé. Selon un aspect plus particulier, le polyélectrolyte anionique réticulé (P1) mis en œuvre dans la composition (C₁) à usage topique objet de la présente invention peut être préparé par la mise en œuvre d'un procédé de polymérisation en milieu solvant suivie d'une étape de précipitation du polymère formé, ou de polymérisation en émulsion inverse optionnellement suivi d'une étape de concentration et/ou d'atomisation.

Selon un aspect plus particulier, le polyélectrolyte anionique réticulé (P1) mis en œuvre dans la composition (C₁) à usage topique objet de la présente invention peut être préparé selon un des procédés décrits ci-dessus et impliquer l'utilisation d'agents de transfert ou limiteurs de chaîne. Les agents de transfert ou limiteurs de chaîne sont plus particulièrement choisi parmi le groupe constitué par l'hypophosphite de sodium, des alcools de faibles poids moléculaires par exemple le méthanol, l'éthanol, le propanol-1, l'isopropanol, le butanol, des thiols, par exemple le 2-mercapto éthanol, des agents de transfert comprenant une fonction sulfate, par exemple le methallylsulfonate de sodium, ou des mélanges desdits agents de transfert. Les agents de transfert ou limiteurs de chaînes sont plus particulièrement utilisés dans des proportions molaires, exprimées par rapport au nombre total de moles de monomères mis en œuvre, de 0,001% à 1% molaire, plus particulièrement de 0,001% à 0,5% molaire, et tout particulièrement de 0,001% à 0,1% molaire.

Selon un autre aspect particulier de la présente invention, ledit polyélectrolyte anionique réticulé (P1) est un élément du groupe constitué par un homopolymère de l'acide acrylique partiellement ou totalement salifié sous forme de sel de sodium ou de sel d'ammonium, réticulé par le triallylamine et/ou le méthylène-bis(acrylamide) ; un homopolymère de l'acide 2-méthyl 2-[(1-oxo 2-propènyl) amino] 1-propanesulfonique partiellement ou totalement salifié sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique partiellement ou totalement salifié sous forme de sel de sodium ou de sel d'ammonium et de l'acide acrylique partiellement ou totalement salifiés sous forme de sel de sodium ou de sel d'ammonium, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium ou de sel d'ammonium et d'acide acrylique (δ) partiellement ou totalement salifié sous forme de sel de sodium dans un rapport molaire (γ)/(δ) supérieur ou égal à 30/70 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acide acrylique (δ) partiellement ou totalement salifié sous forme de sel de sodium dans un rapport molaire (γ)/(δ)supérieur ou égal à 40/60 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acrylamide (ε) dans un rapport molaire (γ)/(ε) supérieur ou égal à 30/70 et inférieur ou égal à 90/10, réticulé par le triallylamine et/ou le méthylène-bis(acrylamide); un copolymère de l'acide 2-méthyl 2-[(1-oxo 2-propènyl) amino] 1-propanesulfonique (γ) partiellement ou totalement salifié sous forme de sel de sodium, et de l'hydroxyéthylacrylate (ζ) dans un rapport molaire (γ)/(ζ) supérieur ou égal à 30/70 et inférieur ou égal à 90/10, réticulé par le triallylamine et/ou le méthylène-bis(acrylamide) ; un terpolymère réticulé par le triallylamine et/ou le méthylènebis(acrylamide), de l'acide 2-méthyl 2-[(1-oxo 2-propènyl) amino] 1-propanesulfonique partiellement ou totalement salifié sous forme de sel de sodium ou de sel d'ammonium, de l'acrylamide et de l'acide acrylique partiellement ou totalement salifiés sous forme de sel de sodium ou de sel d'ammonium ; un terpolymère réticulé par le triallylamine et/ou le méthylène-bis(acrylamide), de l'acide 2-méthyl 2-[(1-oxo 2-propènyl) amino] 1- propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 45% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 10% ; un terpolymère réticulé par le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 30% et inférieure ou égale à 45%, de l'acrylamide dans une proportion molaire supérieure ou égale à 47% et inférieure ou égale à 68% et de l'acide acrylique partiellement ou totalement salifiés sous forme de sel de sodium ou de sel d'ammonium dans une proportion molaire supérieure ou égale à 2% et inférieure ou égale à 8% ; un terpolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39,5% et du méthacrylate de lauryle tétraéthoxylé dans une proportion molaire supérieure ou égale à 0,5% et inférieure ou égale à 5% ; un tétrapolymère réticulé par le trimethylolpropanetriacrylate et/ou le triallylamine et/ou le méthylène-bis(acrylamide), de l'acide 2-méthyl 2-[(1-oxo 2- propènyl) amino] 1-propanesulfonique partiellement ou totalement salifié sous forme de sel de sodium ou de sel d'ammonium dans une proportion molaire supérieure ou égale à 60% et inférieure ou égale à 80%, du N,N diméthylacrylamide dans une proportion molaire supérieure ou égale à 15% et inférieure ou égale à 39 %, du méthacrylate de lauroyle dans une proportion molaire supérieure ou égale à 0,5% et inférieure ou égale à 2,5%, et du méthacrylate de stéaroyle dans une proportion molaire supérieure ou égale à 0,5% et inférieure ou égale à 2,5%.

L'invention a également pour objet une formulation (F) à usage topique comprenant :
- Une composition à usage topique (C1) telle que définie précédemment ; et
- Une phase grasse (A2) comprenant i) au moins une huile, et ii) un système émulsionnant comprenant au moins un agent tensioactif émulsionnant (S1).

De préférence, la formulation selon l'invention comprend pour 100% de sa masse :
- de 60% à 90% massique de la composition (C1) ; et
- de 10% à 40% massique de la phase grasse (A2).

L'agent tensioactif émulsionnant (S1) pourra être sélectionné parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés.

Dans la formulation (F) à usage topique objet de la présente invention, on désigne par « huile » un composé et/ou un mélange de composés insoluble dans l'eau, et liquide à 25°C, et plus particulièrement :
- Les alcanes linéaires comportant de onze à dix-neuf atomes de carbone ;
- Les alcanes ramifiés, comportant de sept à quarante atomes de carbone, comme l'isododécane, l'isopentadécane, l'isohexadécane, l'isoheptadécane, l'isooctadécane, l'isononadécane ou l'isoeicosane), ou des mélanges de certains d'entre eux comme ceux cités ci-après et identifiés par leur nom INCI : C₇₋₈ isoparaffin, C₈₋₉ isoparaffin, C₉₋₁₁ isoparaffin, C₉₋₁₂ isoparaffin, C₉₋₁₃ isoparaffin, C₉₋₁₄ isoparaffin, C₉₋₁₆ isoparaffin, C₁₀₋₁₁ isoparaffin, C₁₀₋₁₂ isoparaffin, C₁₀₋₁₃ isoparaffin, C₁₁₋₁₂ isoparaffin, C₁₁₋₁₃ isoparaffin, C₁₁₋₁₄ isoparaffin, C₁₂₋₁₄ isoparaffin, C₁₂₋₂₀ isoparaffin, C₁₃₋₁₄ isoparaffin, C₁₃₋₁₆ isoparaffin ;
- Les cyclo-alcanes optionnellement substitués par un ou plusieurs radicaux alkyles linéaires ou ramifiés ;
- Les huiles blanches minérales, comme celles commercialisées sous les noms suivants : Marcol™52, Marcol™82, Drakeol™6VR, Eolane™130, Eolane™150 ;
- L'hémisqualane (ou 2,6,10-trimethyl- dodécane ; numéro CAS : 3891-98-3), le squalane (ou 2,6,10,15,19,23-hexamethyltetracosane), le polyisobutène hydrogéné ou le polydécène hydrogéné ;
- Les mélanges d'alcanes comportant de 15 à 19 atomes de carbone, lesdits alcanes étant des alcanes linéaires, des alcanes ramifiés et des cyclo-alcanes, et plus particulièrement le mélange (M₁) qui comprend pour 100% de sa masse, une proportion massique en alcanes ramifiés supérieure ou égale à 90% et inférieure ou égale à 100% ; une proportion massique en alcanes linéaires supérieure ou égale à 0% et inférieure ou égale à 9%, et plus particulièrement inférieure à 5% et une proportion massique en cyclo-alcanes supérieure ou égale à 0% et inférieure ou égale à 1%, par exemple les mélanges commercialisés sous les noms Emogreen™L15 ou Emogreen™L19 ;
- Les éthers d'alcool gras de formule (II) :

   Z₁-O-Z₂ (II),

   dans laquelle Z₁ et Z₂ identiques ou différents, représentent un radical alkyle linéaire ou ramifié comportant de cinq à dix-huit atomes de carbone, par exemple les dioctyl éther, didécyl éther, didodécyl éther, dodécyl octyl éther, dihexadécyl éther, (1,3-diméthyl butyl) tétradécyl éther, (1,3-diméthyl butyl) hexadécyl éther, le bis(1,3-diméthyl butyl) éther ou le dihexyl éther.
- Les mono-esters d'acides gras et d'alcools de formule (III) :

   R'₁-(C=O)-O-R'₂ (III),

   dans laquelle R'₁-(C=O) représente un radical acyle, saturé ou insaturé, linéaire ou ramifié, comportant de huit à vingt-quatre atomes de carbone, et R'2 représente, indépendamment de R'₁, une chaîne hydrocarbonée saturée ou insaturée, linéaire ou ramifiée comportant de un à vingt-quatre atomes de carbone, par exemple les laurate de méthyle, laurate d'éthyle, laurate de propyle, laurate d'isopropyle, laurate de butyle, laurate de 2-butyle, laurate d'hexyle, cocoate de méthyle, cocoate d'éthyle, cocoate de propyle, cocoate d'isopropyle, cocoate de butyle, cocoate de 2-butyle, cocoate d'hexyle, myristate de méthyle, myristate d'éthyle, myristate de propyle, le myristate d'isopropyle, le myristate de butyle, le myristate de 2-butyle, le myristate d'hexyle, le myristate d'octyle, le palmitate de méthyle, le palmitate d'éthyle, le palmitate de propyle, le palmitate d'isopropyle, le palmitate de butyle, le palmitate de 2-butyle, le palmitate d'hexyle, le palmitate d'octyle , l'oléate de méthyle, l' oléate d'éthyle, l'oléate de propyle, l'oléate d'isopropyle, l'oléate de butyle, l'oléate de 2-butyle, l'oléate d'hexyle, l'oléate d'octyle, le stéarate de méthyle, le stéarate d'éthyle, le stéarate de propyle, le stéarate d'isopropyle, le stéarate de butyle, le stéarate de 2-butyle, le stéarate d'hexyle, le stéarate d'octyle, l'isostéarate de méthyle, l'isostéarate d'éthyle, l'isostéarate de propyle, l'isostéarate d'isopropyle, l'isostéarate de butyle, l'isostéarate de 2-butyle, l'isostéarate d'hexyle, l'isostéarate d'isostéaryle ;
- Les di-esters d'acides gras et de glycérol de formule (IV) et de formule (V) :

   R'₃-(C=O)-O-CH₂-CH(OH)-CH₂-O-(C=O)-R'₄ (IV)

   R'₅-(C=O)-O-CH₂-CH[O-(C=O)-R'₆]-CH₂-OH (V),

   formules (VI) (VII) dans lesquelles R'₃-(C=O), R'₄-(C=O), R'₅-(C=O), R'₆-(C=O), identiques ou différents, représentent un groupement acyle, saturé ou insaturé, linéaire ou ramifié, comportant de huit à vingt-quatre atomes de carbone ;
- Les tri-esters d'acides gras et de glycérol de formule (VI) :

   R'₇-(C=O)-O-CH₂-CH[O-(C=O)-R"₈]-CH₂-O-(C=O)-R"₉ (VI),

   dans laquelle R'₇-(C=O), R"₈-(C=O) et R"₉-(C=O), identiques ou différents, représentent un groupement acyle, saturé ou insaturé, linéaire ou ramifié, comportant de huit à vingt-quatre atomes de carbone.
- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- Les huiles végétales éthoxylées.

De préférence, la composition selon l'invention comprend au moins une huile choisie parmi les éléments du groupe constitué par l'huile de ricin, les huiles de paraffine, le cocoyl caprylate/caprate, le myristate d'isopropyle, le capric caprylic triglycéride.

La phase grasse (A2) comprend optionnellement de la cire. Dans la formulation (F) à usage topique objet de la présente invention, on désigne par « cire » un composé et/ou un mélange de composés insoluble dans l'eau, et solide à 35°C

Une telle cire est plus particulièrement choisie parmi la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline; l'ozokérite; la cire de polyéthylène; les cires de silicone ; les cires végétales ; les alcools gras et les acides gras solides à température ambiante; les glycérides solides à température ambiante.

Dans la définition de la formulation (F) objet de la présente invention, on désigne par « compositions d'alkylpolyglycosides », une composition (C₂) représentée par la formule (VII)

R₁-O-(G)ₓ-H (VII)

dans laquelle x représente un nombre décimal compris entre 1,05 et 5, G représente le reste d'un sucre réducteur, et R₁ représente un radical aliphatique hydrocarboné, saturé ou insaturé, linéaire ou ramifié, éventuellement substitué avec un ou plusieurs groupe hydroxyle, comportant de 12 à 36 atomes de carbone, ladite composition (C₂) consistant en un mélange de composés représentés par les formules (VII₁), (VII₂), (VII₃), (VII₄) et (VII₅) :

R₁-O-(G)₁-H (VII₁)

R₁-O-(G)₂-H (VII₂)

R₁-O-(G)₃-H (VII₃)

R₁-O-(G)₄-H (VII₄)

R₁-O-(G)₅-H ( VII₅)

dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5a₅ est égale à x.

Par radical aliphatique hydrocarboné, saturé ou insaturé, linéaire ou ramifié, comportant de 12 à 36 atomes de carbone, éventuellement substitué avec un ou plusieurs groupes hydroxyle, on désigne pour le radical R₁ dans la formule (VII) telle que définie ci-dessus :
- Les radicaux alkyle linéaires saturés, par exemple les radicaux n-dodécyle, n-tridécyle, n-tétradécyle, n-pentadécyle, n-hexadécyle, n-heptadécyle, n-octadécyle, n-nonadécyle, n-eicosyle, n-docosyle ;
- Les radicaux linéaires insaturés tels que les radicaux dodécènyle, tridécènyle, tétradécènyle, pentadécènyle, hexadécènyle, heptadécènyle, octadécènyle, nonadécènyle, eicosènyle, docosènyle, 4-dodécènyle, ou 5-dodécènyle ;
- Les radicaux aliphatiques saturés ou insaturés, linéaires ou ramifiés, comportant de 12 à 36 atomes de carbone substitués par un ou deux groupes hydroxy, tels que les radicaux hydroxydodécyle, hydroxytétradécyle, hydroxyhexadécyle, hydroxyoctadécyle, hydroxyeicosyle, hydroxydocosyle, par exemple le radical 12-hydroxy octadécyle.
- Les radicaux issus des isoalcanols de formule (1) :

   (CH₃)(CH₃)CH-(CH₂)ᵣ-CH₂-OH (1)
dans laquelle r représente un nombre entier compris entre 8 et 20, par exemple les radicaux isodécyle, isoundécyle, isododécyle, isotridécyle, isotétradécyle, isopentadécyle, isohexadécyle, isopentadécyle, isooctadécyle, isononadécyle, isoeicosyle ou isodocosyle ; Les radicaux alkyles ramifiés, issus des alcools de Guerbet, de formule (2) :

CH(CₛH₂ₛ₊₁)(CₜH₂ₜ₊₁)-CH₂-OH (2)

dans laquelle t est un nombre entier compris entre 6 et 18, s est un nombre entier compris entre 4 et 18 et la somme s + t est supérieure ou égale à 10, et inférieure ou égale à 22, par exemple les radicaux 2-butyl octyle, 2-butyl décyle, 2-hexyl octyle, 2-hexyl décyle, 2-octyl décyle, 2-hexyl dodécyle, 2-octyl dodécyle, 2-décyl tétradécyle, 2-dodécyl hexadécyle, 2-tétradécyl octadécyle.

Selon un aspect particulier, dans la définition de la formule (VII) telle que définie ci-dessus, R₁ représente un radical aliphatique hydrocarboné, saturé ou insaturé, linéaire ou ramifié comportant de 12 à 24 atomes de carbone.

Par sucre réducteur, dans la définition de la formule (VII) telle que définie ci-dessus, on désigne les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique (G)ₓ, peut se présenter sous toutes formes d'isoméries, qu'il s'agisse d'isomérie optique, d'isomérie géométrique ou d'isomérie de position ; elle peut aussi représenter un mélange d'isomères.

Dans la formule (VII) telle que définie ci-dessus, le groupe R₁-O- est lié à G par le carbone anomérique du reste saccharide, de manière à former une fonction acétal.

Selon un aspect particulier dans la définition de la formule (VII) telle que définie ci-dessus, G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose; et plus particulièrement G représente le reste d'un sucre réducteur choisi parmi les restes du glucose, du xylose et de l'arabinose.

Selon un aspect encore plus particulier, dans la définition de la formule (VII) représentant la composition (C₂) comprise dans la composition (F) objet de la présente invention, x représente un nombre décimal supérieur ou égal à 1,05 et inférieur ou égal à 2,5, plus particulièrement supérieur ou égal à 1,05 et inférieur ou égal à 2,0, et encore plus particulièrement supérieur ou égal à 1,25 et inférieur ou égal à 2,0.

Selon un aspect encore plus particulier, dans la définition de la formule (VII) telle que définie ci-dessus, R₁ représente le radical choisi parmi au moins un des éléments du groupe constitué par les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle ; G représente le reste d'un sucre réducteur choisi parmi les restes du glucose et du xylose et x représente un nombre décimal supérieur ou égal à 1,05 et inférieur ou égal à 2,5.

Selon un aspect encore plus particulier, la formulation (F) telle que définie précédemment, est caractérisée en ce que ledit système émulsionnant consiste en une composition (C₂) d'alkylpolyglycosides représentée par la formule (VII) :

R₁-O-(G)ₓ-H (VII)

dans laquelle x représente un nombre décimal compris entre 1,05 et 2,5, G représente le reste du xylose, et R₁ représente le radical 2-octyl dodécyle, ladite composition (C₂) consistant en un mélange de composés représentés par les formules (VII₁), (VII₂), (VII₃), (VII₄) et (VII₅) :

R₁-O-(G)₁-H (VII₁)

R₁-O-(G)₂-H (VII₂)

R₁-O-(G)₃-H (VII₃)

R₁-O-(G)₄-H (VII₄)

R₁-O-(G)₅-H (VII₅)

dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5a₅ est égale à x.

Dans la définition de la composition (F) objet de la présente invention, on désigne par composition d'alkylpolyglycosides et d'alcools gras, une composition (C₃) comprenant pour 100% de sa masse :
- De 10% à 50% massique, plus particulièrement de 15% à 40% massique, et encore plus particulièrement de 20% à 30% massique, d'au moins une composition (C₂) représentée par la formule (VII) telle que définie précédemment,
- De 90% à 50% massique, plus particulièrement de 85% à 60% massique, et encore plus particulièrement de 80% à 70% massique, d'au moins un alcool gras de formule (VIII) :

   R'₁-OH (VIII),

   dans laquelle R'₁, identique ou différent de R₁, représente un radical aliphatique hydrocarboné, saturé ou insaturé, linéaire ou ramifié, éventuellement substitué avec un ou plusieurs groupe hydroxyle, comportant de 12 à 36 atomes de carbone.

Selon un aspect particulier, dans la définition de la formule (VII) représentant la composition (C₂) comprise dans la composition (C₃), R₁ représente le radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle, 2-décyl tétradécyle, G représente le reste d'un sucre réducteur choisi parmi les restes du glucose et du xylose et x représente un nombre décimal supérieur ou égal à 1,05 et inférieur ou égal à 2,5.

Selon un aspect plus particulier, dans la définition de la formule (VII) représentant la composition (C₂) comprise dans la composition (C₃), R₁ représente le radical 2-octyl dodécyle, G représente le reste du xylose et x représente un nombre décimal supérieur ou égal à 1,05 et inférieur ou égal à 2,5.

Selon un aspect plus particulier, dans la définition de l'alcool gras de formule (VIII) telle que définie ci-dessus, R'₁ représente un radical choisi parmi les radicaux n-dodécyle, n-tétradécyle, n-hexadécyle, n-octadécyle, n-eicosyle, n-docosyle, 2-hexyl décyle, 2-octyl décyle, 2-hexyl dodécyle, 2-octyl dodécyle ou 2-décyl tétradécyle, R'₁ représente tout particulièrement le radical 2-octyl dodécyle.

Selon un aspect encore plus particulier, l'invention a pour objet une formulation (F) telle que définie précédemment caractérisée en ce que ledit système émulsionnant consiste en une composition (C₃) comprenant pour 100% de sa masse :
- De 10% à 50% massique d'au moins une composition d'alkylpolyglycosides (C₂) représentée par la formule (VII) :

   R₁-O-(G)ₓ-H (VII)

   dans laquelle x représente un nombre décimal compris entre 1,05 et 2,5, G représente le reste du xylose et R₁ représente le radical 2-octyl dodécyle, ladite composition consistant en un mélange de composés représentés par les formules (VII₁), (VII₂), (VII₃), (VII₄) et (VII₅) :

   R₁-O-(G)₁-H (VII₁)

   R₁-O-(G)₂-H (VII₂)

   R₁-O-(G)₃-H (VII₃)

   R₁-O-(G)₄-H (VII₄)

   R₁-O-(G)₅-H (VII₅)

   dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5a₅ est égale à x ; et
- De 90% à 50% massique d'au moins un alcool gras de formule (VIII) :

   R'₁-OH (VIII),

   dans laquelle R'₁ représente le radical 2-octyl dodécyle.

Dans la définition de la formulation (F) objet de la présente invention, on désigne par ester de polyglycérol, un composé de formule (IX) : 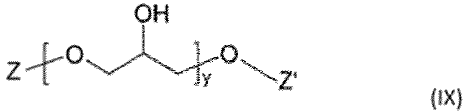 dans laquelle Z représente un radical acyle de formule R₂-C(=O)-, dans laquelle R₂ représente un radical aliphatique hydrocarboné, saturé ou insaturé, linéaire ou ramifié, comportant de 11 à 35 atomes de carbone et plus particulièrement un radical choisi parmi les radicaux dodécanoyle, tétradécanoyle, hexadécanoyle, octadécanoyle, eicosanoyle, docosanoyle, oléyle, linoléyle, linolénoyle ou isostéaryle, Z' représente le radical acyle de formule R₂-C(=O)-tel que défini ci-dessus, avec Z' identique ou différent de Z, ou l'atome d'hydrogène, et y représente un nombre entier supérieur ou égal à 2 et inférieur ou égal à 20.

Selon un aspect plus particulier, le composé de formule (IX) est choisi parmi les éléments du groupe constitué par l'oléate de décaglycérol, l'isostéarate de décaglycérol, le monolaurate de décaglycérol, le mono-linoléate de décaglycérol, le mono-myristate de décaglycérol. Dans la définition de la formulation (F) objet de la présente invention, on désigne par esters de polyglycérols alcoxylés, un composé de formule (X) : 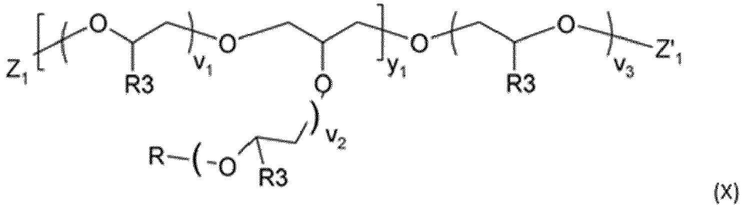 dans laquelle Z₁ représente un radical acyle de formule R'₂-C(=O)-, dans laquelle R'₂ représente un radical aliphatique hydrocarboné, saturé ou insaturé, linéaire ou ramifié, comportant de 11 à 35 atomes de carbone, et plus particulièrement un radical choisi parmi les radicaux radicaux dodécanoyle, tétradécanoyle, hexadécanoyle, octadécanoyle, eicosanoyle, docosanoyle, oléyle, linoléyle, linolénoyle ou isostéaryle, Z₁' représente le radical acyle de formule R'₂-C(=O)- tel que défini ci-dessus, avec Z₁' identique ou différent de Z₁, ou l'atome d'hydrogène, R₃ représente l'atome d'hydrogène, le radical méthyle, ou le radical éthyle, y₁ représente un nombre entier supérieur ou égal à 2 et inférieur ou égal à 20, v₁, v₂, v₃, identiques ou différents, représentent un nombre entier supérieur ou égal à 0 et inférieur ou égal à 50, et la somme [(y₁. v₁) + (y₁. v₂) + v₃)] est un nombre entier supérieur ou égal à 1 et inférieur ou égal à 50.

Dans la définition de la composition (F) objet de la présente invention, on désigne par polyhydroxystéarates de polyglycols, un composé de formule (XI) : 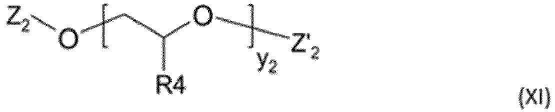 dans laquelle y₂ représente un nombre entier supérieur ou égal à 2 et inférieur ou égal à 50, R₄ représente l'atome d'hydrogène, le radical méthyle, ou le radical éthyle, Z₂ représente un radical de formule (XII) : 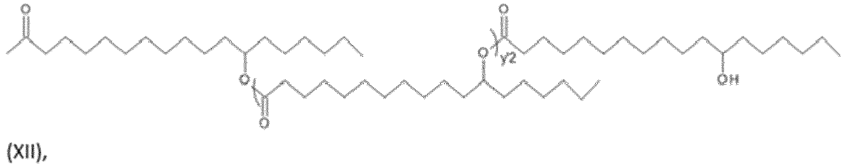 dans laquelle y'₂ représente un nombre entier supérieur ou égal à 0 et inférieur ou égal à 10, plus particulièrement supérieur ou égal à 1 et inférieur ou égal à 10 et Z'₂ représente un radical de formule (XII) telle que définie ci-dessus, avec Z₂' identique ou différent de Z₂, ou l'atome d'hydrogène.

Dans la définition de la formulation (F) objet de la présente invention, on désigne par polyhydroxystéarate de polyglycérol, un composé représenté par la formule (XIII) : 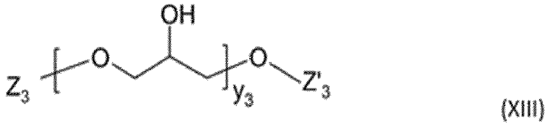 dans laquelle Z₃ représente un radical de formule (XII) telle que définie ci-dessus, Z'₃ représente un radical de formule (XII) telle que définie ci-dessus, avec Z₃' identique ou différent de Z₃, ou l'atome d'hydrogène, y₃ représente un nombre entier supérieur ou égal à 2 et inférieur ou égal à 20.

Dans la définition de la formulation (F) objet de la présente invention, on désigne par polyhydroxystéarate de polyglycérol alcoxylé, un composé représenté par la formule (XIV) : 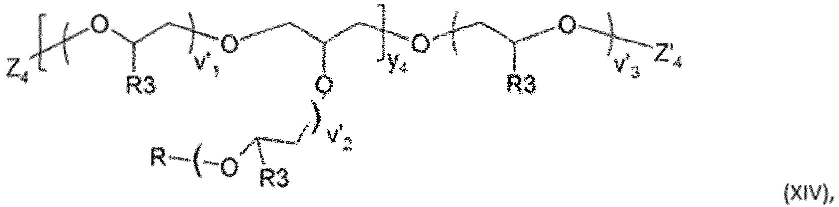 dans laquelle Z₄ représente un radical de formule (XII) telle que définie ci-dessus, Z'₄ représente un radical de formule (XII) telle que définie ci-dessus, avec Z₄' identique ou différent de Z₄, ou l'atome d'hydrogène, y₄ représente un nombre entier supérieur ou égal à 2 et inférieur ou égal à 20, v'₁, v'₂, v'₃, identiques ou différents, représentent un nombre entier supérieur ou égal à 0 et inférieur ou égal à 50, et la somme [(y₄. v'₁) + (y₄. v'₂) + v'₃)] est un nombre entier supérieur ou égal à 1 et inférieur ou égal à 50.

Selon un autre aspect particulier, l'invention a pour objet une formulation (F) telle que définie précédemment, caractérisée en ce que ledit système émulsionnant (S) consiste en une composition (C₄) comprenant pour 100% de sa masse :
De 15% à 25% massique d'au moins une composition (C₂) représentée par la formule (VII) :

R1-O-(G)ₓ-H (VII)

dans laquelle x représente un nombre décimal compris entre 1,05 et 2,5, G représente le reste du xylose et R1 représente le radical 2-octyl dodécyle, ladite composition (C₂) consistant en un mélange de composés représentés par les formules (VII₁), (VII₂), (VII₃), (VII₄) et (VII₅) :

R₁-O-(G)₁-H (VII₁)

R₁-O-(G)₂-H (VII₂)

R₁-O-(G)₃-H (VII₃)

R₁-O-(G)₄-H (VII₄)

R₁-O-(G)₅-H (VII₅)

dans les proportions molaires respectives a₁, a₂, a₃, a₄ et a₅, telles que :
- La somme a₁+ a₂ + a₃ + a₄ + a₅ est égale à 1 et que
- La somme a₁ + 2a₂ + 3a₃ + 4a₄ + 5a₅ est égale à x ;

De 55% à 65% massique d'au moins un alcool gras de formule (VIII) :

R'₁-OH (VIII),

dans laquelle R'₁ représente le radical 2-octyl dodécyle ;

De 10% à 30% massique d'au moins un polyhydroxystéarates de polyglycols représenté par la formule (XI) : 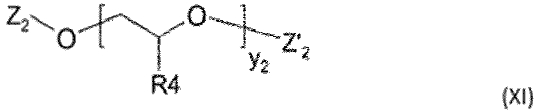 dans laquelle y₂ représente un nombre entier supérieur ou égal à 2 et inférieur ou égal à 50, R₄ représente l'atome d'hydrogène, le radical méthyle ou le radical éthyle, Z₂ représente un radical de formule (XII) : 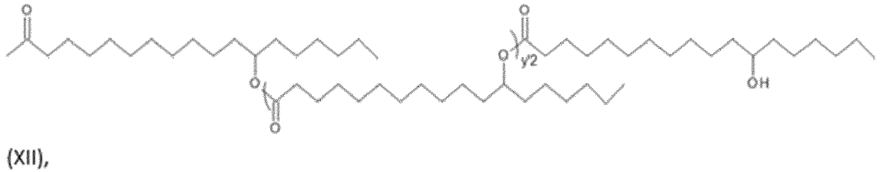 dans laquelle y'₂ représente un nombre entier supérieur ou égal à 0 et inférieur ou égal à 10, plus particulièrement supérieur ou égal à 1 et inférieur ou égal à 10, Z'₂ représente un radical de formule (XII) telle que définie ci-dessus, avec Z₂' identique ou différent de Z₂, ou l'atome d'hydrogène.

Selon un autre aspect particulier, l'invention a pour objet une formulation (F) telle que définie précédemment caractérisée en ce que sa viscosité dynamique de ladite formulation (F), mesurée à une température de 25°C et à l'aide d'un viscosimètre Brookfield de type LVT à une vitesse de 6 tours/minute, est supérieure ou égale à 500 mPa.s et inférieure ou égale à 40 000 mPa.s.

L'invention a aussi pour objet la composition (C1) selon l'invention, administrable par voie topique pour le nettoyage et/ou pour la protection et/ou pour le soin de la peau, des cheveux, du cuir chevelu, des ongles, des lèvres, des cils, des sourcils du corps humain ou animal.

De même, l'invention a aussi pour objet la formulation (F) selon l'invention, administrable par voie topique pour le nettoyage et/ou pour la protection et/ou pour le soin de la peau, des cheveux, du cuir chevelu, des ongles, des lèvres, des cils, des sourcils du corps humain ou animal.

La composition à usage topique (C1) et de la formulation (F) à usage topique telles que définies précédemment peuvent être appliquées sur la peau humaine à l'aide des doigts ou d'un applicateur comme par exemple un pinceau ou une éponge ou une lingette préalablement imprégnée desdites composition à usage topique (C1) et formulation (F) à usage topique telles que définies précédemment.

La composition à usage topique (C1) et la formulation (F) objets de la présente invention peuvent être conditionnée sous forme pressurisée dans un dispositif aérosol ou dans un dispositif de type « flacon pompe », dans un dispositif muni d'une paroi ajourée par exemple une grille, dans un dispositif muni d'un applicateur a billes (dit"roll-on).

La composition à usage topique (C1) et la formulation (F) objets de la présente invention peuvent être utilisées comme laits nettoyants ou démaquillants, comme lotions nettoyantes ou démaquillantes, comme gels moussants pour le visage ou pour le corps, comme shampooing pour le nettoyage des cheveux et/ou du cuir chevelu, comme après-shampooing pour le traitement des cheveux et/ou du cuir chevelu, comme bain moussant, comme crème, comme lait ou comme lotion pour le soin ou pour la protection du visage, des mains et du corps, par exemple comme agent protecteur des rayonnements solaires, comme agent auto-bronzant, comme agent anti-âge, comme agent antirides, comme agent apaisant, comme agent hydratant.

La composition à usage topique (C1) et la formulation (F) objets de la présente invention peuvent en outre comporter des excipients et/ou des principes actifs habituellement mis en œuvre dans le domaine des formulations à usage topique, en particulier cosmétiques, dermocosmétiques, pharmaceutiques ou dermo-pharmaceutique.

C'est pourquoi, selon un autre aspect particulier, l'invention a pour objet une composition à usage topique (C1) et la formulation (F) telles que définies précédemment, caractérisée en ce qu'elle comprennent en outre, un ou plusieurs composés auxiliaires choisis parmi les tensioactifs moussants et/ou détergents, les tensioactifs épaississants et/ou gélifiants, les agents épaississants et/ou gélifiants, les agents stabilisants, les composés filmogènes, les solvants et cosolvants, les agents hydrotropes, les agents plastifiants, les agents émulsionnants et co-émulsionnants, les agents opacificants, les agents nacrants, les agents surgraissants, les séquestrants, les agents chélatants, les agents antioxydants, les parfums, les huiles essentielles, les agents conservateurs, les agents conditionneurs, les agents déodorants, les agents blanchissants destinés à la décoloration des poils et de la peau, les principes actifs destinés à apporter une action traitante et/ou protectrice vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les répulsifs pour les insectes.

Comme exemples de tensioactifs moussants et/ou détergents, éventuellement présents dans la composition à usage topique (C1) et dans la formulation (F) objets de la présente invention, on peut citer les tensioactifs moussants et/ou détergents anioniques, cationiques, amphotères ou non ioniques topiquement acceptables habituellement utilisés dans ce domaine d'activité.

Parmi les tensioactifs anioniques moussants et/ou détergents que l'on peut associer à la composition à usage topique (C1) et à la formulation (F) objets de la présente invention, on peut citer les sels de métaux alcalins, les sels de métaux alcalino-terreux, les sels d'ammonium, les sels d'amines, les sels d'amino alcools d'alkyléthers sulfates, d'alkyl sulfates, d'alkylamidoéther sulfates, d'alkylarylpolyéther sulfates, de monoglycérides sulfates, d'alpha-oléfinesulfonates, de paraffines sulfonates, d'alkyl phosphates, d'alkyléther phosphates, d'alkyl sulfonates, d'alkylamide sulfonates, d'alkylaryl sulfonates, d'alkyl carboxylates, d'alkylsulfosuccinates, d'alkyléther sulfosuccinates, d'alkylamide sulfosuccinates, d'alkyl sulfo-acétates, d'alkyl sarcosinates, d'acyliséthionates, de N-acyl taurates, d'acyl lactylates, de dérivés N-acylés d'acides aminés, de dérivés N-acylés de peptides, de dérivés N-acylés de protéines, d'acides gras.

Parmi les tensioactifs amphotères moussants et/ou détergents que l'on peut associer à la composition à usage topique (C1) et à la formulation (F) objets de la présente invention, on peut citer les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques moussants et/ou détergents éventuellement présents dans la composition à usage topique (C1) et dans la formulation (F) objets de la présente invention, on peut citer particulièrement les dérivés d'ammoniums quaternaires.

Parmi les tensioactifs non ioniques moussants et/ou détergents éventuellement présents dans la composition à usage topique (C1) et dans la formulation (F) objets de la présente invention, on peut citer plus particulièrement les dérivés d'huile de ricin, les polysorbates, les amides de coprah, les N-alkylamines.

Comme exemples de tensioactifs épaississants et/ou gélifiants éventuellement présents dans la composition à usage topique (C1) et dans la formulation (F) objets de la présente invention, on peut citer:
- - les esters gras d'alkylpolyglycosides éventuellement alcoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120;
- les esters gras alcoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141 ;
- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples de tensioactifs émulsionnants éventuellement présents dans la composition à usage topique (C1) et dans la formulation (F) objets de la présente invention, on peut citer des tensioactifs non ioniques, des tensioactifs anioniques, des tensioactifs cationiques.

Comme exemples de tensioactifs non ioniques émulsionnants éventuellement présents dans la composition à usage topique (C1) et dans la formulation (F) objets de la présente invention, on peut citer les esters d'acides gras et de sorbitol, par exemple les produits commercialisés sous les appellations MONTANE™80 et MONTANE™85 et MONTANE™60; l'huile de ricin éthoxylée et l'huile de ricin hydrogénée éthoxylée, par exemple le produit commercialisé sous la dénomination SIMULSOL™ 989; les compositions comprenant du stéarate de glycérol et de d'acide stéarique poly(éthoxylé) avec entre 5 moles et 150 moles d'oxyde d'éthylène, par exemple la composition comprenant de l'acide stéarique (éthoxylé) à 135 moles d'oxyde d'éthylène et du stéarate de glycérol commercialisée sous l'appellation SIMULSOL™ 165; les esters de sorbitan éthoxylés, par exemple les produits commercialisés sous la dénomination MONTANOX™ ; les esters de mannitan ; les esters de mannitan éthoxylés ; les esters de sucrose ; les esters de méthylglucoside.

Comme exemples de tensioactifs anioniques émulsionnants éventuellement présents dans la composition à usage topique (C1) et dans la formulation (F) objets de la présente invention, on peut citer le décylphosphate, le cétylphosphate commercialisé sous l'appellation AMPHISOL™, le glycéryl stéarate citrate ; le cétéarylsulfate ; la composition arachidyl/béhényl phosphates et arachidyl/béhényl alcools commercialisée sous l'appellation SENSANOV™WR; les savons par exemple le stéarate de sodium ou le stéarate de triéthanolammonium, les dérivés N-acylés d'acides aminés salifiés comme par exemple le stéaroyl glutamate.

Comme exemples de tensioactifs cationiques émulsionnants éventuellement présents dans la composition à usage topique (C1) et dans la formulation (F) objets de la présente invention, on peut citer les aminoxydes, le quaternium-82 et les tensioactifs décrits dans la demande de brevet WO96/00719 et principalement ceux dont la chaîne grasse comprend au moins 16 atomes de carbone.

Comme exemples d'agents opacifiants et/ou nacrants éventuellement présents dans la composition à usage topique (C1) et dans la formulation (F) objets de la présente invention, on peut citer le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras comportant de 12 à 22 atomes de carbone.

Comme exemples d'agents de texture éventuellement présents dans la composition à usage topique (C1) et dans la formulation (F) objets de la présente invention, on peut citer des dérivés N-acylés d'acides aminés, par exemple la lauroyl lysine commercialisée sous l'appellation AMINOHOPE™LL, l'amidon de babassu commercialisé sous le nom de marque SEPIFINE™, l'octenyl starch succinate commercialisé sous l'appellation DRYFLO™, le myristyl polyglucoside commercialisé sous l'appellation MONTANOV 14, les fibres de cellulose, les fibres de coton, les fibres de chitosane, le talc, la séricite, le mica.

Comme exemples de solvants et de co-solvants éventuellement présents dans la composition à usage topique (C1) et dans la formulation (F) objets de la présente invention, on peut citer l'eau, les solvants organiques par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, les alcools hydrosolubles tels que l'éthanol, l'isopropanol ou le butanol, les mélanges d'eau et desdits solvants organiques, le propylène carbonate, l'acétate d'éthyle, l'alcool benzylique.

Comme exemples d'agents d'amélioration de la pénétration cutanée éventuellement présents dans la composition à usage topique (C1) et dans la formulation (F) objets de la présente invention, on peut citer les glycols éthers comme par exemple l'éthylène glycol mono méthyl éther, l'éthylène glycol mono éthyl éther, l'éthylène glycol mono propyl éther, l'éthylène glycol mono isopropyl éther, l'éthylène glycol mono butyl éther, l'éthylene glycol mono phényl éther, l'éthylène glycol mono benzyl éther, le diéthylène glycol mono méthyl éther, le diéthylène glycol mono éthyl éther et le diéthylène glycol mono-n-butyl éther, le diéthylène glycol mono éthyléther (ou Transcutol-P), les acides gras comme l'acide oléique, les esters de glycérol d'acide gras comme par exemple le béhénate de glycérol, le palmitostéarate de glycérol, le béhénoyl macroglycérides, le polyoxyéthylène-2-stéaryl éther, le polyoxyéthylène-2-oléyl éthers, des terpènes comme par exemple le D-Limonène, des huiles essentielles comme par exemple l'huile essentielle d'eucalyptus.

Comme exemples d'agents déodorants éventuellement présents dans la composition à usage topique (C1) et dans la formulation (F) objets de la présente invention, on peut citer les silicates alcalins, les sels de zinc comme le sulfate de zinc, le gluconate de zinc, le chlorure de zinc, le lactate de zinc; les sels d'ammonium quaternaires comme les sels de cétyltriméthylammonium, les sels de cétylpyridinium ; les dérivés du glycérol comme le caprate de glycérol , le caprylate de glycérol, le caprate de polyglycérol ; le 1,2-décanediol ; le 1,3-propanediol ; l'acide salicylique ; le bicarbonate de sodium; les cyclodextrines ; les zéolithes métalliques; le Triclosan™; le bromohydrate d'aluminium, les chlorhydrates d'aluminium, le chlorure d'aluminium, le sulfate d'aluminium, les chlorhydrates d'aluminium et de zirconium, le trichlorhydrate d'aluminium et de zirconium, le tétrachlorhydrate d'aluminium et de zirconium, le pentachlorhydrate d'aluminium et de zirconium, l'octochlorhydrate d'aluminium et de zirconium, le sulfate d'aluminium, le lactate de sodium et d'aluminium, les complexes de chlorhydrate d'aluminium et de glycol, comme le complexe de chlorhydrate d'aluminium et de propylène glycol, le complexe de dichlorhydrate d'aluminium et de propylène glycol, le complexe de sesquichlorhydrate d'aluminium et de propylène glycol, le complexe de chlorhydrate d'aluminium et de polyéthylène glycol, le complexe de dichlorhydrate d'aluminium et de polyéthylène glycol, le complexe de sesquichlorhydrate d'aluminium et de polyéthylène glycol.

Comme exemples d'agents anti-oxydants éventuellement présents dans la composition à usage topique (C1) et dans la formulation (F) objets de la présente invention, on peut citer l'EDTA et ses sels, l'acide citrique, l'acide tartrique, l'acide oxalique, le BHA (butylhydroxyanisol), le BHT (butylhydroxytoluène), les dérivés de tocophérol tels que l'acétate de tocophérol, des mélanges de composés antioxydants tels que la DISSOLVINE GL 47S (nom INCI: Tetrasodium Glutamate Diacetate).

La composition (C1) et la formulation (F) selon l'invention peuvent comprendre un agent biologiquement actif.

Parmi les principes actifs que peuvent comprendre la composition à usage topique (C1) et dans la formulation (F) objets de la présente invention, on peut citer :
- les vitamines et leurs dérivés, notamment leurs esters, tels que le rétinol (vitamine A) et ses esters (comme par exemple palmitate de rétinyle), l'acide ascorbique (vitamine C) et ses esters, les dérives de sucre de l'acide ascorbique (comme l'ascorbyl glucoside), le tocophérol (vitamine E) et ses esters (comme l'acétate de tocophérol), les vitamines B3 ou B10 (niacinamide et ses dérivés);
- les composés montrant une action apaisante notamment le SEPICALM™ S, l'allantoïne, le bisabolol, le produit commercialisé sous le nom de marque TECA™ (extrait de *Centella asiatica) ;*
- les agents anti-inflammatoires comme par exemple les agents anti-inflammatoires non stéroïdiens (ou AINS), et plus particulièrement les dérivés arylacétiques (ou arylalkanoïques) et les acides 2-arylpropioniques (ou profènes), et en core plus particulièrement le diclofénac, l'acide tiaprofénique, l'alminoprofène, l'étodolac, le flurbiprofène, l'ibuprofène, le kétoprofène , le naproxène ;
- les agents analgésiques et anti-inflammatoires et plus particulièrement l'acétaminophène, l'aspirine, l'acide salicylique, le méthyl salicylate, la choline salicylate, le glycol salicylate, le 1-menthol, le camphre, l'acide méfénamique, l'acide fluphénamique, l'indométhacine, l'acide protizidique, le fentiazac, le tolmetin, l'acide tiaprofenique, le phénylbutazone, l'oxyphenbutazone, le clofezone, le pentazocine, le mepirizole, l'hydrocortisone, la cortisone, la dexamethasone, la fluocinolone, la triamcinolone, la medrysone, la prednisolone, la flurandrenolide, le prednisone, l'halcinonide, le méthylprédnisolone, le fludrocortisone, la corticosterone, la paramethasone, la betamethasone ;
- les agents antiseptiques et plus particulièrement la cetrimide, la povidone-iodine, la chlorhexidine, l'iodine, le benzalkonium chloride, l'acide benzoique, le nitrofurazone, le péroxyde de benzoyle, l'hydrogène péroxyde, l'hexachlorophène, le phénol, le résorcinol et le cétylpyridinium chloride ;
- les agents insecticides et plus particulièrement le trichlorfone, le triflumerone, le fenthion, le bendiocarbe, la cyromazine, le dislubenzurone, le dicyclanile, le fluazurone, l'amitraze, la deltamethrine, la cypermethrine, le chlorfenbinphose, la flumethrine, la ivermectine, l'abermectine, l'avermectine, la doramectine, la moxidectine, la zeti-cypermethrine, la diazinone, la spinosade, l'imidaclopride, le nitenpyrane, le pyriproxysene, le sipronil, le cythioate, la lufenurone, la selamectine, la milbemycine oxime, le chlorpyrifose, le coumaphose, le propetamphose, l'alpha-cypermethrine, l'highciscypermethrine, l'ivermectine, la diflubenzurone, le cyclodiene, le carbamate and benzoyl urée ;
- les agents anti-microbiens et plus particulièrement les sulfonamides, les aminoglycosides comme par exemple le neomycine, le tobramycine, le gentamycine, l'amikacine, le kanamycine, le spectinomycine, le paromomycine, le netilmicin, les polypeptides, les cephalosporines, les oxazolidinones comme par exemple la ciprofloxacine, la levofloxacine, l'ofloxacine ;
- les composés montrant une action hydratante comme l'urée, les hydroxy-urées, le glycérolglucoside, le diglycérolglucoside, les polyglycérylglucosides, le glycérol, le diglycérol, le xylitylpolyglucoside commercialisé sous le nom de marque Aquaxyl™, le maltitol, le sorbitylpolyglucoside, l'acide hyaluronique et ses sels comme par exemple le hyaluronate de sodium, le hyaluronate de potassium et plus particulièrement un l'acide hyaluronique ou un sel d'acide hyaluronique dont le poids moléculaire est compris entre 20 kDa et 5000 kDa, plus particulièrement entre 500 kDa et 2200 kDa ;
- les extraits végétaux riches en polyphénols comme les extraits de raisin, les extraits de pin, les extraits de vin, les extraits d'olives;
- les composés montrant une action amincissante ou lipolytique comme la caféine ou ses dérivés, l'ADIPOSUM™, l'ADIPOLESS™, la fucoxanthine;
- les protéines N-acylées, les peptides N-acylés comme le MATRIXIL™; les acides aminés N-acylés; les hydrolysâts partiels de protéines N-acylés; les acides aminés; les peptides; les hydrolysâts totaux de protéines;
- les extraits végétaux comme par exemple les extraits de soja, par exemple la Raffermine™, les extraits de blé comme par exemple la TENSINE™ ou la GLIADINE™, les extraits végétaux, tels que les extraits végétaux riches en tanins, les extraits végétaux riches en isoflavones ou les extraits végétaux riches en terpènes; les extraits d'algues d'eau douce ou marines, les extraits de plantes marines; les extraits marins en général comme les coraux; les cires essentielles; les extraits bactériens;
- les céramides;
- les phospholipides;
- les composés montrant une action antimicrobienne ou une action purifiante, comme le LIPACIDE™ C8G, le LIPACIDE™ UG, le SEPICONTROL™ A5; l'OCTOPIROX™ ou le SENSIVA™ SC50;
- les composés montrant une propriété énergisante ou stimulante comme le PHYSIOGENYL™, le panthénol et ses dérivés comme le SEPICAP™ MP;
- les actifs anti-âge comme le SEPILIFT™ DPHP, le LIPACIDE™ PVB, le SEPIVINOL™, le SEPIVITAL™, le MANOLIVA™, le PHYTO-AGE™, le TIMECODE™; le SURVICODE™;
- les actifs anti-photo vieillissement; les actifs protecteurs de l'intégrité de la jonction dermo-épidermique; les actifs augmentant la synthèse des composants de la matrice extracellulaire comme le collagène, les élastines, les glycosaminoglycanes; les actifs agissant favorablement sur la communication cellulaire chimique comme les cytokines ou physiques comme les intégrines;
- les actifs créant une sensation de « chauffe » sur la peau comme les activateurs de la microcirculation cutanée (comme les dérivés de l'acide nicotinique) ou des produits créant une sensation de « fraîcheur » sur la peau (comme le menthol et des dérivés);
- les actifs améliorant la microcirculation cutanée, par exemple les veinotoniques; les actifs drainants; les actifs à visée décongestionnante comme les extraits de ginko biloba, de lierre, de marron d'inde, de bambou, de ruscus, de petit houx, de centalla asiatica, de fucus, de romarin, de saule;
- les agents de bronzage ou de brunissement de la peau, comme par exemple la dihydroxyacétone (DHA), l'érythrulose, l'aldéhyde mésotartrique, le glutaraldéhyde, le glycéraldéhyde, l'alloxane, la ninhydrine, les extraits végétaux comme par exemple les extraits de bois rouges du genre Pterocarpus et du genre Baphia comme le Pteropcarpus santalinus, le Pterocarpus osun, le Pterocarpus soyauxii, le Pterocarpus erinaceus, le Pterocarpus indicus ou le Baphia nitida comme ceux décrits dans la demande de brevet Européen EP 0 971683 ;
- les agents connus pour leur action de facilitation et/ou d'accélération du bronzage et/ou du brunissement de la peau humaine, et/ou pour leur action de coloration de la peau humaine, comme par exemple les caroténoïdes (et plus particulièrement le beta carotène et le gamma carotène), le produit commercialisé sous le nom de marque « Carrot oil » (Nom INCI: Daucus Carota, helianthus annuus Sunflower oil) par la société Provital, qui contient des caroténoïdes, de la vitamine E et de la vitamine K; la tyrosine et/ou ses dérivés, connus pour leur effet sur l'accélération du bronzage de la peau humaine en association avec une exposition aux rayonnements ultra-violets, comme par exemple le produit commercialisé sous le nom de marque « SunTan Accelerator™ » par la société Provital qui contient de la tyrosine et des riboflavines (vitamine B), le complexe de tyrosine et de tyrosinase commercialisé sous le nom de marque « Zymo Tan Complex » par la société Zymo Line, le produit commercialisé sous le nom de marque MelanoBronze™ (nom INCI: Acetyl Tyrosine, Monk's pepper extract (Vitex Agnus-castus)) par la société Mibelle qui contient de l'acétyl tyrosine, produit commercialisé sous le nom de marque Unipertan VEG-24/242/2002 (nom INCI: butylene glycol and Acetyl Tyrosine and hydrolyzed vegetable protein and Adenosine triphosphate) par la société UNIPEX, le produit commercialisé sous le nom de marque « Try-Excell™ » (nom INCI: Oleoyl Tyrosine and Luffa Cylindrica (Seed) Oil and Oleic acid) par la société Sederma qui contient des extraits de pépins de courge (ou huile de Loofah), le produit commercialisé sous le nom de marque «Actibronze™ » (nom INCI: hydrolyzed wheat protein and acetyl tyrosine and copper gluconate) par la société Alban Muller, le produit commercialisé sous le nom de marque Tyrostan™ (nom INCI: potassium caproyl tyrosine) par la société Synerga, le produit commercialisé sous le nom de marque Tyrosinol (nom INCI: Sorbitan Isostearate, glyceryl oleate, caproyl Tyrosine) par la société Synerga, le produit commercialisé sous le nom de marque InstaBronze™ (nom INCI: Dihydroxyacetone and acetyl tyrosine and copper gluconate) commercialisé par la société Alban Muller, le produit commercialisé sous le nom de marque Tyrosilane (nom INCI: méthylsilanol and acétyl tyrosine) par la société Exymol; les peptides connus pour leur effet d'activation de la mélanogénèse comme par exemple le produit commercialisé sous le nom de marque Bronzing SF Peptide powder (nom INCI: Dextran and Octapeptide-5) par la société Infinitec Activos, le produit commercialisé sous le nom de marque Melitane (nom INCI: Glycerin and Aqua and Dextran and Acetyl hexapeptide-1) comprenant l'acétyl hexapeptide-1 connu pour son action agoniste de l'alpha-MSH, le produit commercialisé sous le nom de marque Melatimes Solutions™ (nom INCI: Butylene glycol, Palmitoyl Tripeptide-40) par la société LIPOTEC, les sucres et les dérivés de sucres comme par exemple le produit commercialisé sous le nom de marque Tanositol™ (nom INCI: inositol) par la société Provital, le produit commercialisé sous le nom de marque Thalitan™ (ou Phycosaccharide™ AG) par la société CODIF international (nom INCI: Aqua and hydrolyzed algin (Laminaria Digitata) and magnesium sulfate and manganese sulfate) contenant un oligosaccharide d'origine marine (acide guluronique et acide mannuronique chélatés avec les ions magnésium et manganèse), le produit commercialisé sous le nom de marque Melactiva™ (nom INCI: Maltodextrin, Mucuna Pruriens Seed extract) par la société Alban Muller, les composés riches en flavonoïdes comme par exemple le produit commercialisé sous le nom de marque « Biotanning » (nom INCI: Hydrolyzed citrus Aurantium dulcis fruit extract) par la société Silab et connu pour être riche en flavonoides de citron (de type hespéridines).

Comme exemples de filtres solaires éventuellement présents dans la composition à usage topique (C1) et dans la formulation (F) objets de la présente invention, on peut citer tous ceux figurant dans la directive cosmétique 76/768/CEE modifiée annexe VII.

Parmi les filtres organiques solaires éventuellement présents dans la composition à usage topique (C1) et dans la formulation (F) objets de la présente invention, on peut citer :
- Les dérivés de l'acide benzoïque comme les acides para-aminobenzoïques (PABA), notamment les esters de monoglycérol de PABA, les esters éthyliques de N,N-propoxy PABA, les esters éthyliques de N,N-diéthoxy PABA, les esters éthyliques de N,N-diméthyl PABA, les esters méthyliques de N,N-diméthyl PABA, les esters butyliques de N,N-diméthyl PABA;
- Les dérivés de l'acide anthranilique comme l'homomenthyl-N-acétyl anthranilate ;
- Les dérivés de l'acide salicylique comme le salicylate d'amyle, le salicylate d'homomenthyle, le salicylate d'éthylhexyle, le salicylate de phényle, le salicylate de benzyle, le salicylate de p-isopropanolphényle ;
- Les dérivés de l'acide cinnamique comme le cinnamate d'éthylhexyle, le cinnamate d'éthyl-4-isopropyle, le cinnamate de méthyl-2,5-diisopropyle, le cinnamate de p-méthoxypropyle, le cinnamate de p-méthoxyisopropyle, le cinnamate de p-méthoxyisoamyle, le cinnamate de p-méthoxyoctyle (le cinnamate de p-méthoxy 2-éthylhexyle), le cinnamate de p-méthoxy 2-éthoxyéthyle, le cinnamate de p-méthoxycyclohexyle, le cinnamate d'éthyl-α-cyano-β-phényle, le cinnamate de 2-éthylhexyl-α-cyano-β-phényle, le cinnamate de diparaméthoxy mono-2-éthylhexanoyl de glycéryle ;
- Les dérivés de la benzophénone comme la 2,4-dihydroxybenzophénone, la 2,2'-dihydroxy-4-méthoxybenzophénone, la 2,2',4,4'-tétrahydroxybenzophénone, la 2-hydroxy-4-méthoxybenzophénone, la 2-hydroxy-4-méthoxy-4'-méthylbenzophénone, la 2-hydroxy-4-méthoxybenzophénone-5-sulfonate, la 4-phénylbenzophénone, le 2-éthylhexyl-4'-phénylbenzophénone-2-carboxylate, la 2-hydroxy-4-n-octyloxybenzophénone, la 4-hydroxy-3-carboxybenzophénone ; le 3-(4'-méthylbenzylidène)-d,l-camphre, le 3-(benzylidène)-d,l-camphre, le benzalkonium méthosulfate camphre ; l'acide urocanique, l'urocanate d'éthyle ; la famille des dérivés de l'acide sulfonique comme l'acide sulfonique 2-phénylbenzimidazole-5 et ses sels ;
- Les dérivés de la triazine comme l'hydroxyphényl triazine, l'(éthylhexyloxyhydroxyphényl) (4-méthoxy phényl) triazine, le 2,4,6-trianillino (p-carbo-2'-éthylhexyl-1'-oxy)-1,3,5-triazine, le benzoate de 4,4-((6-(((1,1-diméthyléthyl) amino)carbonyl)phenyl)amino)-1,3,5-triazine-2,4-diyl diimino) bis-(2-éthylhexyl), le 2-phényl-5-méthylbenzoxazole, le 2,2'-hydroxy-5-méthylphénylbenzotriazole, le 2-(2'-hydroxy-5'-t-octylphényl)benzotriazole, le 2-(2'-hydroxy-5'-méthyphényl)benzotriazole; la dibenzazine; le dianisoylméthane, le 4-méthoxy-4"-t-butylbenzoylméthane ; la 5-(3,3-diméthyl-2-norbornylidène)-3-pentan-2-one ;
- Les dérivés du diphénylacrylate comme le 2-éthylhexyl-2-cyano-3,3-diphényl-2-propènoate, l'éthyl-2-cyano-3,3-diphényl-2-propènoate ;
- Les polysiloxanes comme le malonate de benzylidène siloxane.

Parmi les filtres inorganiques solaires, également appelés "écrans minéraux", éventuellement présents dans la composition à usage topique (C1) et dans la formulation (F) objets de la présente invention, on peut citer les oxydes de titane, les oxydes de zinc, l'oxyde de cérium, l'oxyde de zirconium, les oxydes de fer jaune, rouge ou noir, les oxydes de chrome. Ces écrans minéraux peuvent être micronisés ou non, avoir subi ou non des traitements de surface et être éventuellement présentés sous formes de pré-dispersions aqueuses ou huileuses.

Le procédé de préparation de la composition (C₁) telle que définie précédemment, comprend les étapes suivantes:
- Une étape a) de préparation de la phase aqueuse par introduction dans une cuve de volume appropriée, de l'eau minérale naturelle ou de l'eau thermale ou de l'eau de mer ou de l'eau d'algues à une température de 25°C ; de l'eau déminéralisée peut être introduite pour atteindre par dilution la valeur de la conductivité souhaitée, qui sera supérieure ou égale à 300 µS.cm⁻¹,
- Une étape b) d'ajout, sous agitation mécanique appropriée et à 25°C, du polyélectrolyte anionique réticulé (P1) sur la phase aqueuse précédemment préparée et maintenir sous agitation jusqu'à l'observation d'une gélification du milieu, et
- Une étape c) d'ajout, sous agitation mécanique appropriée et à 25°C, du galactomannane (GM) sur la phase aqueuse gélifiée précédemment préparée et maintenir sous agitation jusqu'à l'observation d'un gel homogène.

Le procédé de préparation de la formulation (F) telle que définie précédemment, comprend les étapes suivantes:
- Une étape a) de préparation de la phase grasse (A₂) en mélangeant l'ensemble des éléments la constituant dans les proportions souhaitées. Cette étape de mélange est généralement conduite à une température supérieure ou égale à 25°C et inférieure ou égale à 80°C, plus particulièrement supérieure ou égale à 25°C et inférieure ou égale à 60°C, et encore plus particulièrement supérieure ou égale à 25°C et inférieure ou égale à 40°C ; elle est réalisée sous agitation mécanique à une vitesse modérée supérieure ou égale à 50 tours/minute et inférieure ou égale à 100 tours/minute ;
- Une étape b) de préparation de la composition (C₁) telle que précédemment décrite ; de façon particulière, la phase aqueuse (A₁) obtenue à l'issue de l'étape b), présente une viscosité dynamique, mesurée à 25°C par l'intermédiaire d'un viscosimètre de type Brookfield LV à une vitesse de 6 tours/minute, supérieure ou égale à 200 mPa.s et inférieure ou égale à 40 000 mPa.s, plus particulièrement supérieure ou égale à 1 000 mPa.s et inférieure ou égale à 40 000 mPa.s, et encore plus particulièrement supérieure ou égale à 2 000 mPa.s et inférieure ou égale à 40 000 mPa.s ;
- Une étape c) au cours de laquelle la phase grasse (A₂) est ajoutée sur la composition (C₁) à une température supérieure ou égale à 25°C et inférieure ou égale à 80°C, plus particulièrement supérieure ou égale à 25°C et inférieure ou égale à 60°C, et encore plus particulièrement supérieure ou égale à 25°C et inférieure ou égale à 40°C, sous agitation mécanique à une vitesse modérée supérieure ou égale à 50 tours/minute et inférieure ou égale à 400 tours/minute, de façon à obtenir la formulation (F).

Les exemples suivants illustrent l'invention, sans toutefois la limiter.

### Préparation et évaluation de gels aqueux selon l'invention et de gels aqueux comparatifs.

### 1) Préparation des gels aqueux

On prépare quatre gels aqueux selon l'invention, notés (F₁) à (F₄), et huit gels aqueux comparatifs, notées (F'₁) à (F'₈), dont les proportions massiques en leurs constituants sont consignées dans le tableau 1 ci-dessous, en mettant en œuvre le procédé ci-après.

La phase aqueuse est préparée par introduction à 25°C d'une eau à conductivité élevée et d'une eau déminéralisée, dans des proportions relatives telles qu'elles permettent d'obtenir une phase aqueuse à conductivité recherchée (par exemple 730 µ/cm ou 3,28 mS/cm dans les éléments expérimentaux ci-dessous. Le polyélectrolyte anionique réticulé étudié est ensuite progressivement introduit à 25°C sur l'eau préalablement préparée sous agitation mécanique modérée, à savoir avec un mobile d'agitation de type ancre et à une vitesse comprise entre 50 et 150 tours/min, jusqu'à l'obtention d'un gel homogène. Le galactomannane (GM) étudié est ajouté sur le gel précédemment formé à une température de 25°C sous agitation avec un agitateur mécanique équipé d'un mobile de type ancre. Cette agitation est alors maintenue pendant dix minutes et aucune étape de refroidissement n'est nécessaire.

### 2) Evaluation des propriétés des gels aqueux

On contrôle les propriétés des gels aqueux par :
- l'évaluation de l'aspect des gels préparés, en recherchant un aspect visuel homogène du gel à 25°C, et
- la mesure de la viscosité dynamique des gels préparés à 25°C

Les résultats des caractérisations et des mesures sont indiqués dans le tableau 1 ci-dessous.

**[Table 1]**

| | **(F₁)** | **(F'₁)** | **(F'₂)** | **(F'₃)** | **(F'₄)** | **(F'₅)** |
|---|---|---|---|---|---|---|
| **Eau** | | | | | | |
| Eau à conductivité (σ₁) égale à 3,3µs/cm) à 25°C | 88,5% | | 88,5% | 88,5% | 88,5% | 88,5% |
| Hydralixir™ LD⁽¹⁾ à conductivité (σ₂) égale à 6,07mS/cm à 25°C | 10% | 10% | 10% | 10% | 10% | 10% |
| Conductivité totale de l'eau (σ_{T} = σ₁ + σ₂) à 25°C | 730 µs/cm | 730 µs/cm | 730 µs/cm | 730 µs/cm | 730 µs/cm | 730 µs/cm |
| Solagum™ AX ⁽²⁾ | 0% | 0% | 0,5% | 0% | 0% | 0% |
| Solagum™Tara⁽³⁾ | 0,5% | 0% | 0% | 0,5% | 1% | 1,5% |
| Sepinov™ EMT 10 ⁽⁴⁾ | 1,5% | 1,5% | 1,5% | 0% | 0% | 0% |
| SlMUlGEL™EG^{(s)(*)} | 0% | 0% | 0% | 0% | 0% | 0% |
| pH à 25°C | 5,47 | 5,56 | 5,35 | 5,72 | 5,66 | 5,69 |
| Viscosité (J1) BKF RVT M7 V5 à 25°C | 190000 mPa.s | 48000 mPa.s | 41 100 mPa.s | 240 mPa.s | 5 000 mPa.s | 41 000 mPa.s |
| Aspect visuel à 25°C | Homogène et coulable | Homogène et coulable | Homogène et coulable | Hétérogène et Observation de dépôts | Hétérogène et Observation de dépôts | Homogène et coulable |

**Tableau 1 : constitution, caractérisation gel aqueux (F1) selon l'invention et des gels aqueux comparatifs (F'1) (F'2) (F'3) (F'4) (F'5)**

| | **(F₂)** | **(F₃)** | **(F₄)** | **(F'₆)** | **(F'₇)** | **(F'₈)** |
|---|---|---|---|---|---|---|
| **Eau** | | | | | | |
| Eau déminéralisée à conductivité (σ₁) égale à 3,3µs/cm à 25°C | 86,15% | 86,15% | 48% | 86,65% | 86,65% | 48,5% |
| Hydralixir™ LD⁽¹⁾ à conductivité (σ₂) égale à 6,07ms/cm à 25°C | 10% | 10% | 50% | 10% | 10% | 50% |
| Conductivité totale de l'eau(σᵣ=σ₁+ σ₂) à 25°C | 730µS/cm | 730µS/cm | 3,28mS/cm | 730 µS/cm | 730µS/cm | 3,28 mS/cm |
| Solagum™Tara⁽³⁾ | 0,5% | 0,5% | 0,5% | 0% | 0% | 0% |
| Sepinov™ EMT 10⁽⁴⁾ | 0% | 0% | 1,5% | 0% | 0% | 1,5% |
| SIMULGEL™EG^{(5)(*)} | 3,35% | 0% | 0% | 3,35% | 0% | 0% |
| SEPIGEL™305 ^{(6)(**)} | 0% | 3,35% | 0% | 0% | 3,35% | 0% |
| pH à 25°C | 5,75 | 5,75 | 5,56 | 5,68 | 5,57 | 5,72 |
| Viscosité (J1) BKF RVT M7V5 à 25°C | 170 000 mPa.s | 220 000 mPa.s | 43 600 mPa.s | 61 000mPa.s | 115 000 mPa.s | 2 340 mPa.s |
| Aspect visuel à 25°C | Homogène et compact | Homogène et coulable | Homogène et compact | Hétérogène et compact | Hétérogène et compact | Homogène et coulable |

Tableau 1 (suite) : constitution, caractérisation des gels aqueux (F2), (F3), et (F4) selon l'invention et des gels aqueux comparatifs (F'6), (F'7) et (F'8)
(1) Hydralixir™ LD (nom INCI : water and Laminaria Digitata Extract) est une eau cellulaire d'algues brunes, obtenue à partir de la Laminaria Digitata.
(2) Solagum™ AX (nom INCI : acacia Senegal gum and Xanthan gum) est un agent épaississant et stabilisant pour la préparation de compostions cosmétiques
(3) Solagum™ Tara (nom INCI : Caesalpinia Spinosa Gum) est un galactomannane, de degré de substitution de 1/3, utilisé agent épaississant et stabilisant pour la préparation de compostions cosmétiques.
(4) Sepinov™ EMT 10 (nom INCI : hydroxyethyl Acrylate/Sodium Acryloyldimethyl Taurate Copolymer) est un agent épaississant, stabilisant et émulsionnant, se présentant sous la forme d'une poudre et utilisé pour la preparation de compositions cosmétiques
(5) Simulgel™EG (nom INCI : Sodium Acrylate/Sodium Acryloyldimethyl Taurate Copolymer & Isohexadecane & Polysorbate 80) est un agent épaississant, stabilisant et émulsionnant, se présentant sous la forme d'un latex inverse auto-inversible, utilisé pour la préparation de compositions cosmétiques
(6) : Sepigel™305 (nom INCI : Polyacrylamide (and) C13-14 Isoparaffin (and) Laureth-7) est un agent épaississant, stabilisant et émulsionnant, se présentant sous la forme d'un latex inverse auto-inversible, utilisé pour la préparation de compositions cosmétiques.
(*) : le Simugel™EG se présente sous la forme d'un latex inverse auto-inversible, et sa teneur en en polyélectrolyte anionique réticulée est de 40%
(**) : le Sepigel™305 se présente sous la forme d'un latex inverse auto-inversible, et sa teneur en en polyélectrolyte anionique réticulée est de 40%

### 3) Analyse des résultats

L'utilisation de la gomme de Tara, à une teneur massique de 0,5%, en association avec 1,5% massique du polyélectrolyte anionique réticulé commercialisé sous le nom de marque Sepinov™EM10, permet d'obtenir un gel aqueux (F₁) de très forte viscosité (190 000 mPa.s pour (F₁)) alors qu'un gel aqueux obtenu avec la proportion massique de 1,5% de Sepinov™EMT 10 (F'₁) ne permet d'atteindre qu'une viscosité de 48 000 mPa.s.

L'obtention de gels aqueux de forte viscosité n'est pas observée lorsque la gomme de Tara est remplacée par un mélange de gomme d'acacia et de gomme de xanthane : la viscosité du gel comparatif (F'₂) obtenu est de 41100 mPa.s, soit très proche du niveau de viscosité du gel aqueux (F'₁) obtenu avec le seul polyélectrolyte anionique réticulé commercialisé sous le nom de marque Sepinov™EM10.

Les viscosités des gels comparatifs (F'₃), (F'₄) et (F'₅), obtenus avec la seule gomme de Tara à différentes doses (respectivement 0,5%, 1% et 1,5%), montrent des valeurs respectives de 240 mPa.s, 5 000 mPa.s et 41 000 mPa.s, ne laissant ainsi pas présumer du niveau de viscosité obtenu par association de 0,5% de gomme de Tara et de 1,5% massique du polyélectrolyte anionique réticulé commercialisé sous le nom de marque Sepinov™EM10. De telles viscosités élevées s'observent également lorsque la gomme de Tara est associée à un polyélectrolyte anionique réticulé dont le squelette polymérique est de nature différente : copolymère réticulé à base d'acrylate de sodium et d'acryloyldiméthyl taurate de sodium (170 000 mPa.s pour le gel aqueux (F₂) selon l'invention), ou copolymère réticulé à base d'acrylamide et d'acryloyldiméthyl taurate de sodium (220 000 mPa.s pour le gel aqueux (F₃) selon l'invention).

Par ailleurs, la comparaison de la viscosité du gel aqueux (F₄) selon l'invention avec la viscosité du gel aqueux comparatif (F'₈) démontre également un effet synergique de la combinaison lorsque la conductivité de la phase aqueuse est plus élevée (3,28 mS/cm pour (F₄) et (F'₈)).

## Revendications

1. Composition à usage topique (C1) se présentant sous la forme d'un gel et comprenant:
- de l'eau dont la conductivité mesurée à 25°C est supérieure ou égale à 300 micro-siemens par centimètre (ou µS.cm⁻¹)
- un polyélectrolyte anionique réticulé (P1) et
- au moins un galactomannane (GM).

2. Composition (C1) selon la revendication 1, **caractérisé en ce que** la composition comprend pour 100% de sa masse :
- De 93% à 99,4% massique de l'eau dont la conductivité mesurée à 25°C est supérieure ou égale à 300 micro-siemens par centimètre (ou µS.cm⁻¹)
- De 0,5 à 5% massique du polyélectrolyte anionique réticulé (P1) et
- De 0,1 à 2% massique du galactomannane (GM).

3. Composition (C1) selon l'une des revendications 1 ou 2, **caractérisée en ce que** le galactomannane présente un degré de substitution d'1/3.

4. Composition (C1) selon l'une des revendications 1 à 3, **caractérisée en ce que** le polyélectrolyte anionique réticulé (P1) comprend des unités monomériques issues de l'acide 2-méthyl 2-[(1-oxo 2-propènyl) amino] 1-propanesulfonique sous forme acide libre ou partiellement ou totalement salifiée.

5. Composition (C1) selon l'une des revendications 1 à 4, **caractérisée en ce que** le polyélectrolyte anionique réticulé (P1) est constitué de :
- une unité monomérique issue d'au moins un monomère choisi parmi les éléments du groupe constitué par l'acide acrylique, l'acide méthacrylique, l'acide 2-carboxyéthyl acrylique, l'acide itaconique, l'acide maléique, l'acide 3-méthyl 3-[(1-oxo 2-propènyl) amino] butanoïque, la fonction carboxylique desdits monomères étant sous forme acide libre, partiellement salifiée ou totalement salifiée ; et
- au moins une unité monomérique issue d'un monomère de réticulation (AR) polyéthylénique.

6. Composition (C1) selon la revendication 5, **caractérisée en ce que** le polyélectrolyte anionique réticulé (P1) est constitué d'une unité monomérique issue de l'acide acrylique et d'au moins une unité monomérique issue d'un monomère de réticulation (AR) polyéthylénique.

7. Composition (C1) selon l'une des revendications 5 ou 6, **caractérisée en ce que** le monomère de réticulation (AR) polyéthylénique est choisi parmi les éléments du groupe constitué par le méthylène-bis(acrylamide), le diméthacrylate d'éthylèneglycol, le diacrylate de diéthylèneglycol, le diacrylate d'éthylène glycol, le diallyl urée, le triallylamine, le triméthylol propanetriacrylate, l'acide diallyoxyacétique ou un de ses sels comme le diallyloxyacétate de sodium, ou un mélange de ces composés.

8. Composition (C1) selon l'une des revendications 5 à 7, **caractérisée en ce que** le monomère de réticulation (AR) polyéthylénique est le triallylamine, le triméthylol propanetriacrylate ou le méthylène-bis(acrylamide) ou un mélange ces composés.

9. Composition (C1) selon l'une des revendications 1 à 8, **caractérisée en ce que** ledit polyélectrolyte anionique réticulé (P1) est un élément du groupe constitué par un copolymère de l'acide 2-méthyl 2-[(1-oxo 2-propènyl) amino] 1-propanesulfonique (γ) partiellement ou totalement salifié sous forme de sel de sodium, et de l'acide acrylique (δ) partiellement ou totalement salifié sous forme de sel de sodium dans un rapport molaire (γ)/(δ)supérieur ou égal à 40/60 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'hydroxyéthylacrylate (ζ) dans un rapport molaire (γ)/(ζ) supérieur ou égal à 30/70 et inférieur ou égal à 90/10, réticulé par le triallylamine et/ou le méthylène-bis(acrylamide) ; un copolymère de l'acide 2-méthyl 2-[(1-oxo 2-propènyl) amino] 1-propanesulfonique (γ) partiellement ou totalement salifié sous forme de sel de sodium, et de l'acrylamide (ε) dans un rapport molaire (γ)/(ε) supérieur ou égal à 30/70 et inférieur ou égal à 90/10, réticulé par le triallylamine et/ou le méthylène-bis(acrylamide).

10. Composition (C1) selon l'une des revendications 1 à 9, **caractérisée en ce que** le ratio massique entre le galactomannane (GM) et le polyélectrolyte anionique réticulé (P1) est supérieur ou égal à 0,15 et inférieur ou égal à 0,75.

11. Formulation (F) à usage topique comprenant :
- Une composition à usage topique (C1) telle que définie à l'une des revendications 1 à 10; et
- Une phase grasse (A2) comprenant i) au moins une huile, et ii) un système émulsionnant comprenant au moins un agent tensioactif émulsionnant (S1).

12. Formulation selon la revendication 11, **caractérisée en ce qu'**elle comprend pour 100% de sa masse :
- De 60% à 90% massique de la composition (C1) ; et
- De 10% à 40% massique de la phase grasse (A2).

13. Formulation selon l'une des revendications 11 ou 12, **caractérisée en ce que** l'agent tensioactif émulsionnant (S1) est sélectionné parmi les éléments du groupe constitué par les compositions d'alkylpolyglycosides, les compositions d'alkylpolyglycosides et d'alcools gras, les esters de polyglycérols, les esters de polyglycérols alcoxylés, les polyhydroxystéarates de polyglycols, les polyhydroxystéarates de polyglycérols, les polyhydroxystéarates de polyglycérols alcoxylés.

14. Composition (C1) selon l'une des revendications 1 à 10, administrable par voie topique pour le nettoyage et/ou pour la protection et/ou pour le soin de la peau, des cheveux, du cuir chevelu, des ongles, des lèvres, des cils, des sourcils du corps humain ou animal.

15. Formulation (F) selon l'une des revendications 11 à 13, administrable par voie topique pour le nettoyage et/ou pour la protection et/ou pour le soin de la peau, des cheveux, du cuir chevelu, des ongles, des lèvres, des cils, des sourcils du corps humain ou animal.

16. Composition (C1) selon l'une des revendications 1 à 10, **caractérisée en ce qu'**elle comprend un agent biologiquement actif.

17. Formulation (F) selon l'une des revendications 11 à 13, **caractérisée en ce qu'**elle comprend un agent biologiquement actif.
